(19) Europäisches Patentamt
European Patent Office
Office européen des brevets

(11) **EP 2 134 249 B1**

(12) **FASCICULE DE BREVET EUROPEEN**

(45) Date de publication et mention
de la délivrance du brevet:
**20.02.2013 Bulletin 2013/08**

(51) Int Cl.:
*A61B 3/113* (2006.01)    *A61B 3/14* (2006.01)
*G02C 13/00* (2006.01)    *A61B 3/11* (2006.01)

(21) Numéro de dépôt: **08787855.9**

(22) Date de dépôt: **26.03.2008**

(86) Numéro de dépôt international:
**PCT/FR2008/000412**

(87) Numéro de publication internationale:
**WO 2008/132356 (06.11.2008 Gazette 2008/45)**

(54) **PROCÉDÉ DE MESURE DE LA POSITION SUIVANT UNE DIRECTION HORIZONTALE DU PLAN SAGITTAL DU CENTRE DE ROTATION D'UN OEIL D'UN SUJET**

VERFAHREN ZUR MESSUNG DER POSITION DES DREHPUNKTS IM AUGE EINER PERSON AUF DER HORIZONTALEN SAGITTALEBENE

METHOD FOR MEASURING THE POSITION OF THE CENTER OF ROTATION OF THE EYE OF A SUBJECT ALONG THE HORIZONTAL DIRECTION OF THE SAGITTAL PLANE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **30.03.2007 FR 0702335**

(43) Date de publication de la demande:
**23.12.2009 Bulletin 2009/52**

(73) Titulaire: **Essilor International (Compagnie Générale d'Optique) 94220 Charenton-le-Pont (FR)**

(72) Inventeur: **CHAUVEAU, Jean-Pierre F-94220 Charenton Le Pont (FR)**

(74) Mandataire: **Chauvin, Vincent et al Coralis 14/16, rue Ballu 75009 Paris (FR)**

(56) Documents cités:
**EP-A- 1 747 750        WO-A- 02/09025
WO-A-2006/029875    WO-A-2006/106248
DE-A1-102004 020 356   DE-A1-102005 003 699
US-A- 3 454 331        US-B1- 6 580 448**

EP 2 134 249 B1

## Description

DOMAINE TECHNIQUE AUQUEL SE RAPPORTE L'INVENTION

La présente invention concerne de manière générale la prise de mesures géométrico-morphologiques sur un sujet. Elle vise plus particulièrement un procédé de mesure de la position d'un point remarquable de l'oeil du sujet. Elle trouve une application particulière, mais non exclusive, à la prise de mesures géométrico-morphologiques sur futur porteur de lunettes, en vue de la conception optique personnalisée de lentilles ophtalmiques correctrices à monter dans la monture choisie par ce futur porteur.

ARRIÈRE-PLAN TECHNOLOGIQUE

Lors de la conception optique d'une lentille ophtalmique correctrice, on cherche actuellement à prendre mieux en compte des paramètres géométrico-morphologiques individuels, dits de conception optique personnalisée, attachés au porteur et à la monture qu'il a choisie. Ces paramètres comprennent notamment la configuration spatiale de la lentille par rapport à la tête du porteur, dans les conditions du porté. Cette configuration spatiale est déterminée par (i) l'orientation de la lentille par rapport à l'oeil correspondant du porteur et (ii) la distance entre la lentille et l'oeil correspondant du porteur.
Pour déterminer cette configuration spatiale, l'opticien place une paire de lunettes de présentation sur le nez du porteur. Les lunettes de présentation comportent la monture choisie par le porteur et des lentilles non correctrices montées dans les cercles de la monture.
La mesure de la distance entre chaque lentille et l'oeil correspondant du porteur est effectuée manuellement : l'opticien observe le porteur de profil et réalise une mesure à l'estime, au moyen d'un réglet, de la distance entre la cornée et la face arrière de la lentille de présentation.
On a proposé d'automatiser cette mesure en photographiant de profil le porteur et en traitant l'image ainsi acquise pour tenter de déterminer la distance séparant la lentille de l'oeil. Cependant, l'opération de traitement d'image s'est avérée peu fiable dans la mesure où les cercles et branches de la monture peuvent masquer l'oeil et où la reconnaissance du point de la face arrière de la lentille en regard de l'oeil s'avère périlleuse. En outre, la prise de vue de profil du porteur s'ajoute à d'autres mesures géométrico-morphologiques sur porteur, telles que la mesure des écarts interpupillaires et hauteurs des yeux, du galbe de la monture, de l'angle pantoscopique de chaque lentille au porté (angle que forme le plan général de la lentille par rapport à la verticale), ou encore le comportement visuel du porteur (en particulier sa tendance à bouger plus ou moins les yeux ou la tête lorsqu'il scrute le champ visuel). Or, tout ou partie de ces autres mesures peuvent être effectuées au moyen d'une ou plusieurs prises de vue de face du porteur. La demanderesse s'est donc fixé pour objectif, dans le cadre de la présente invention, de proposer une méthode permettant d'effectuer une mesure de la distance entre l'oeil et la lentille à partir de prises de vue globalement de face, et non de profil.
Par ailleurs, la distance séparant la pupille de la face arrière de la lentille n'est pas la grandeur la plus pertinente pour réaliser un calcul de design optique personnalisé par tracés de rayon de la lentille correctrice destinée à équiper la monture. En effet, le mouvement de l'oeil est globalement assimilable à une combinaison de rotations autour d'un point particulier appelé Centre de Rotation de l'Oeil (CRO). C'est la position de ce point que le concepteur de la lentille souhaite connaître pour effectuer convenablement son calcul. Selon !a pratique actuelle, la position du CRO est déduite de façon approximative de celle de la cornée en considérant une valeur moyenne de rayon de l'oeil, typiquement d'environ 15 mm. Or, le rayon d'oeil varie sensiblement d'un individu à l'autre, si bien que cette approximation conduit à des erreurs importantes qui pénalisent lourdement la pertinence du calcul optique personnalisé.
Un appareil de mesure du centre de rotation de l'oeil est connu du document WO 2006/106248 A1 qui décrit un procédé et dispositif pour la détermination du centre de rotation d'un oeil (CRO) d'une personne par rapport à un repère lié à la personne ou à sa paire de lunettes, permettant la détermination de l'axe visuel de cette personne dans au moins deux directions non parallèles au moyen d'une cible visualisée et la définition d'un point optimal dit de croisement de ces axes comme centre de rotation de l'oeil, au moins deux positions relatives de cette cible (2) et de la tête de la personne étant mesurées. Ladite cible est constituée d'une source de lumière et est disposée à une extrémité d'un support tubulaire (3) dont l'autre extrémité est destinée à être disposée en face de l'oeil de la personne, ce support portant un premier capteur de position (4), un second capteur de position (7) étant destiné à être placé sur la tête de la personne.
D'autres procédés de mesure du mouvement des yeux sont connus des document DE 10 2004 020 356 A1, EP 1 747 750 A1 et WO 02/09025 A1.

OBJET DE L'INVENTION

Le but de la présente invention est de remédier à tout ou partie des inconvénients précités.
A cet effet, on propose selon l'invention un procédé de mesure de la position, suivant une direction horizontale du plan sagittal, d'un point remarquable d'un oeil d'un sujet dans un référentiel lié à la tête de ce sujet, conforme à la revendication 1.

Le rapprochement des images des premier et second points de référence de l'oeil est représentatif du déplacement angulaire apparent de l'oeil partir de deux points de vue différents correspondant aux première et seconde postures relatives. Un calcul de parallaxe permet alors, compte-tenu des informations sur les deux points de vue que constituent les première et seconde valeurs du paramètre de posture, d'obtenir la position recherchée.

De plus, le procédé selon l'invention peut être mis en oeuvre à l'aide d'un unique appareil de capture d'image.

Enfin, le calcul du paramètre de posture correspondant à chaque posture du sujet est réalisé à partir des seules images planes capturées de face dans les différentes postures, sans que d'autres mesures soient effectuées.

D'autres caractéristiques avantageuses et non limitatives du procédé selon l'invention sont énoncées dans les revendications 2 à 14.

Selon encore une autre caractéristique avantageuse de l'invention, lors des étapes S1) et S4), on agence la tête du sujet par rapport à la pupille d'entrée de l'appareil de capture d'image, dans les première et seconde postures relatives, de manière que la position relative de la pupille de l'appareil de capture d'image par rapport à un axe de rotation vertical de la tête du sujet ne soit pas modifiée entre lesdites première et seconde postures par un déplacement transversal de plus de 200 millimètres suivant une direction perpendiculaire à l'axe optique de l'appareil de capture d'image, et de manière que le sujet pivote la tête autour dudit axe de rotation vertical d'au moins 5 degrés et d'au plus 60 degrés entre lesdites première et seconde postures pour fixer du regard respectivement des premier et second points de visée ayant des positions distinctes connues l'une par rapport à l'autre.

Cette combinaison de caractéristiques résout le problème technique additionnel expliqué ci-dessous, qui concerne l'obtention de deux postures relatives différentes de la tête du sujet par rapport à la pupille d'entrée de l'appareil de capture d'image.

Pour que la tête du sujet soit agencée dans deux postures relatives différentes par rapport à la pupille d'entrée de l'appareil de capture d'image, on peut procéder de différentes manières.

Si l'appareil de capture d'image est mobile, on peut déplacer cet appareil de capture d'image entre les première et seconde captures d'image alors que l'axe de rotation vertical de la tête sujet reste immobile. La tête du sujet pivote autour de cet axe de rotation vertical pour que les yeux du sujet fixent la pupille de l'appareil de capture d'images lorsque celui-ci se déplace. Dans ce cas, l'opticien doit s'assurer que l'ensemble du visage du porteur est visible sur l'image capturée par l'appareil de capture d'image et que les ajustements de hauteur et d'inclinaison relatives entre la tête du porteur et l'appareil de capture d'images sont bien réalisés. Ces opérations limitent la rapidité d'exécution des captures d'image ainsi que leur précision.

Si l'appareil de capture d'image présente une position identique lors des première et seconde captures d'image, l'opticien peut demander au sujet de se déplacer tout en fixant du regard la pupille d'entrée de cet appareil de capture d'image. Les consignes de positionnement données par l'opticien au sujet assurent que le visage du porteur reste visible sur chaque image capturée par l'appareil de capture d'image. Les interactions complexes et fastidieuses entre le sujet et l'opticien limitent cependant la rapidité d'exécution des première et seconde captures d'image.

Les deux postures relatives de la pupille de l'appareil de capture d'image et de la tête du sujet sont obtenues de façon plus simple et plus rapide si la position relative de la pupille de l'appareil de capture d'image et de l'axe de rotation vertical de la tête du sujet reste sensiblement identique lors des première et seconde captures d'image et que seule la tête du sujet tourne autour de cet axe de rotation vertical tout en fixant des première et seconde positions d'un point de visée de l'appareil de capture d'images.

Les consignes de positionnement données par l'opticien au sujet, et plus généralement les interactions entre l'opticien et le sujet, sont alors limitées. De plus, l'opticien peut effectuer une capture d'image avec une meilleure précision, puisqu'il n'a pas à ajuster successivement entre deux captures d'image la hauteur ou l'inclinaison relative entre le sujet et l'appareil de capture d'image, ou bien plus généralement le cadrage pour que la tête du sujet reste visible sur l'image capturée.

DESCRIPTION DÉTAILLÉE D'UN EXEMPLE DE RÉALISATION

La description qui va suivre, en regard des dessins annexés, donnée à titre d'exemple non limitatif, fera bien comprendre en quoi consiste l'invention et comment elle peut être réalisée.

Sur les dessins annexés :

- la figure 1 est une vue en perspective d'une paire de lunettes de présentation disposée devant les yeux d'un futur porteur ;
- la figure 2 est une vue en perspective d'un système de repérage destiné à équiper la paire de lunettes de présentation ;
- la figure 3 est une vue schématique de l'image des lunettes de présentation et du système de repérage capturée de face ;

- la figure 4 est une vue en perspective du système de repérage de la figure 2 fixé sur la paire de lunettes de présentation et des moyens de capture d'image qui communiquent avec un système de traitement et de calcul ;
- la figure 5 est une vue de profil de la tête d'un porteur équipé des lunettes de présentation dans une configuration orthostatique ;
- la figure 6 est une vue de dessus du porteur équipé des lunettes de présentation dans une posture où il tourne la tête d'un certain angle par rapport à l'appareil de capture d'image ;
- la figure 7 est une vue de dessus de la monture équipée des lentilles de présentation ;
- la figure 8 est une vue en plan de principe, de dessus, du système de repérage et des moyens de capture d'image ;
- la figure 9 est une autre vue en plan de principe, de dessus, du système de repérage et des moyens de capture d'image, dans une première posture ;
- la figure 10 est une vue analogue à la précédente dans une seconde posture ;
- la figure 11 est une vue analogue à la précédente dans une troisième posture, avec une source iumineuse visibie additionnelle formant un point de visée connu pour le porteur.

Dans la description qui suit, l'opticien détermine la configuration du référentiel de chaque lentille ophtalmique correctrice (non représentée) à monter dans une monture 10 de lunettes dans un référentiel lié à la tête du porteur et dans la configuration du porté. Cette configuration est utilisée pour mettre en oeuvre un procédé de conception optique personnalisée de la lentille ophtalmique correctrice en calculant des géométries de l'une et/ou l'autre des faces optiquement utiles de la lentille et/ou des gradients d'indice de la lentille en fonction du référentiel de la lentille par rapport au référentiel du porteur.

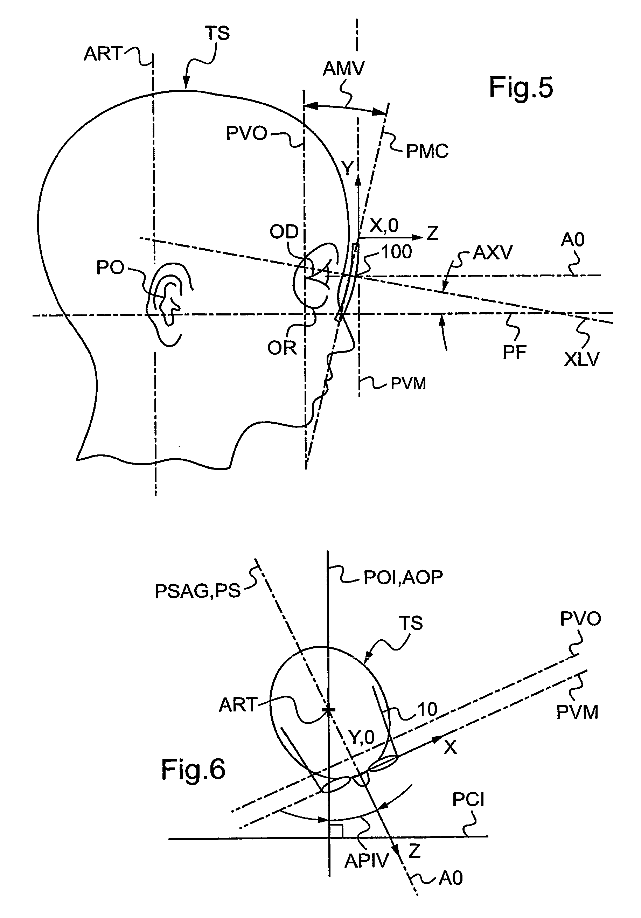

Le porteur est dans une configuration assise ou debout qui est telle que la tête du porteur est droite, c'est-à-dire que le plan de Francfort PF relatif à la tête du porteur est sensiblement horizontal. Comme représenté sur la figure 5, le plan de Francfort PF est défini comme le plan passant par les points orbitaires inférieurs OR et le porion PO du porteur, le porion étant le point du crâne le plus élevé du conduit auditif, qui correspond au tragion de l'oreille. L'axe de regard du porteur est l'axe de regard primaire, c'est-à-dire qu'il regarde l'horizon droit devant lui. On dit également que le porteur prend une position orthostatique, position dans laquelle il réalise un minimum d'efforts.

On définit un plan sagittal PSAG comme étant le plan vertical passant par la médiatrice AO des deux yeux OG, OD. La médiatrice AO des yeux est l'axe passant au milieu du segment défini par les centres de rotation CROG, CROD des deux yeux et parallèle au plan de Francfort PF. On définit aussi un plan vertical oeil PVO comme étant le plan vertical reliant les centres CROG, CROD des yeux.

La configuration recherchée du référentiel de chaque lentille correctrice est déterminée par :

- l'orientation de chaque lentille correctrice à monter sur la monture par rapport à la tête du porteur et
- la distance, dans la configuration du portée, entre la lentille et l'oeil correspondant du porteur suivant une direction horizontale du plan sagittal PSAG (direction de l'axe Z qui est défini plus loin).

Le référentiel de chaque lentille correctrice est obtenu ici à partir de la détermination de la configuration du référentiel de chacune des lentilles de présentation 100, 101 non correctrices qui équipe, pour la vente et les prises de mesure, la monture et qui se substitue, pour la détermination de paramètres géométrico-morphologiques relatifs au porteur et à la monture, à la lentille correctrice à concevoir.

La paire de lunettes de présentation comporte la monture 10 choisie par le porteur et des lentilles de présentation droite et gauche 100, 101 (non correctrices). Dans l'exemple illustré, la paire de lunettes est de type cerclé, c'est-à-dire que les lentilles sont montées dans les cercles 11, 12 de la monture 10. En variante, la paire de lunettes de présentation peut être de type percé, c'est-à-dire que les lentilles sont percées et maintenues chacune par une extrémité du pontet nasal et une extrémité de la branche associée à la lentille, qui coopèrent avec des trous de perçage.

Dispositif

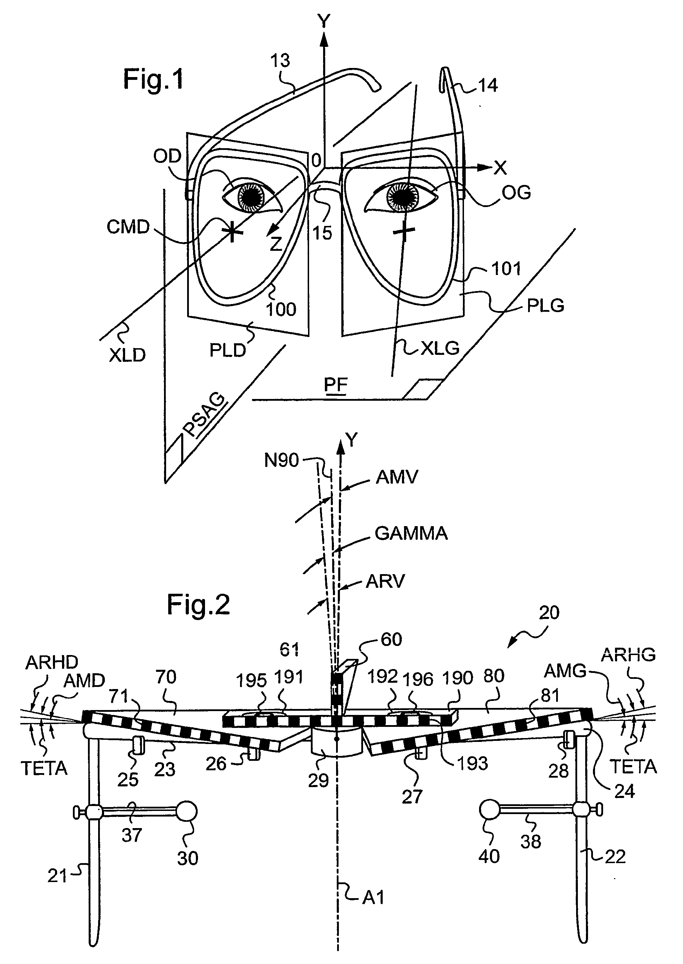

Aux figures 2 à 4, on a représenté un dispositif de détermination de paramètres géométrico-morphologiques individuels d'un porteur équipé d'une paire de lunettes de présentation. Ces paramètres comportent la configuration du référentiel de chaque lentille correctrice à concevoir par rapport à la tête du porteur, ainsi que d'autres paramètres géométrico-morphologiques de répartition de gradients d'indice évoqués plus loin.

Ce dispositif comporte un système de repérage 20 à monter sur la monture 10 et des moyens de capture d'image 90 pour capturer dans un plan facial de capture d'image PCI, l'image du système de repérage 20 monté sur la monture 10 en position de porté. Les moyens de capture d'image 90 sont reliés à un système de traitement et de calcul 93 de l'image capturée.

Le système de repérage 20 comporte une ossature articulée comportant deux baguettes 23, 24 sensiblement rectilignes et sensiblement coplanaires, reliées entre elles par une charnière 29 présentant un axe d'articulation A1 sensiblement vertical dans la configuration du porté.

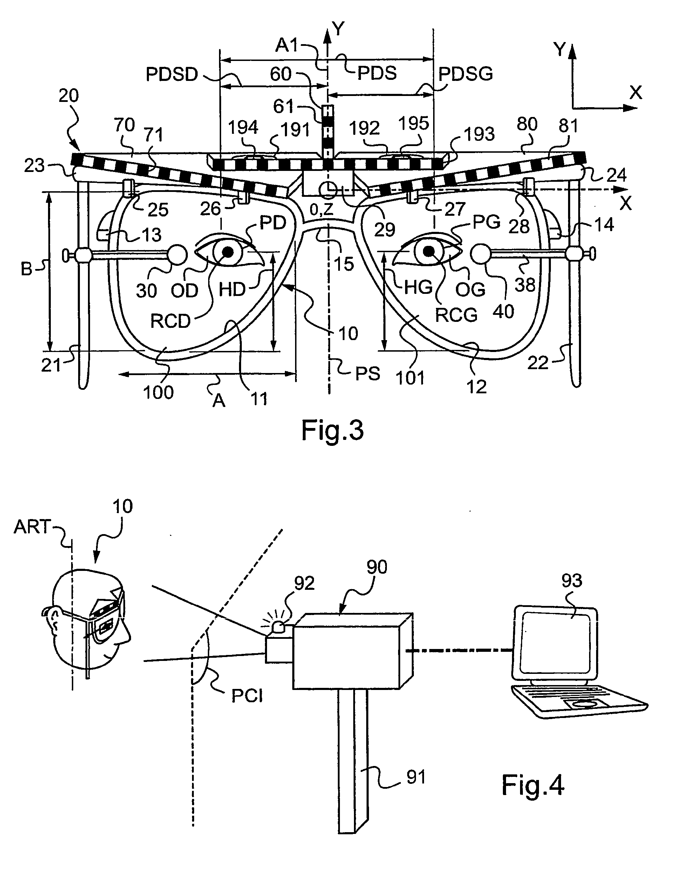

Chaque baguette 23, 24 est munie de moyens de fixation qui se présentent ici sous la forme d'une paire de pinces 25, 26, 27, 28. Ces pinces permettent de fixer, avec une capacité de pivotement, chaque baguette 23, 24 sur la partie supérieure sensiblement horizontal du cercle 11, 12 de la monture (figure 3) ou, lorsque les lunettes sont du type percées, de la lentille de présentation.

Chaque baguette 23, 24 est surmontée d'un élément de repérage horizontal 70, 80 qui se présente sous la forme d'une plaque triangulaire, d'une certaine épaisseur, dont une tranche présente une figure géométrique 71, 81 conçue de telle sorte que la configuration géométrique de cette figure géométrique 71, 81 projetée dans ledit plan facial de capture d'image PCI soit représentative de la composante horizontale de l'orientation de cet élément de repérage horizontal 70, 80. La composante horizontale de l'orientation d'un élément est définie par l'angle que fait la direction longitudinale de cet élément par rapport au plan vertical oeil PVO en projection dans le plan de Francfort PF. De même, la composante verticale de l'orientation d'un élément est définie par l'angle que fait la direction longitudinale de cet élément par rapport au plan vertical oeil PVO en projection dans le plan sagittal PSAG.

Ici, la figure géométrique comporte des motifs répétés d'espacement connu qui sont constitués par des bandes sombres, alternés avec des portions claires pour assurer un contraste suffisant. Les motifs de la figure géométrique 71, 81 s'étendent selon la direction longitudinale de la tranche correspondante de l'élément de repérage horizontal 70, 80. Chaque bande sombre est donc sensiblement verticale dans la configuration du porteur.

Chaque élément de repérage horizontal 70, 80 est fixé sur la baguette 23, 24 correspondante de telle sorte que, d'une part, la tranche portant la figure géométrique 71, 81 soit visible de face et, d'autre part, la direction d'étendue de cette figure géométrique (c'est-à-dire la direction longitudinale de la tranche correspondante) forme, dans le plan horizontal ou plan de Francfort PF, un angle TETA d'environ 30 degrés avec la direction longitudinale de la baguette 23, 24 (c'est-à-dire la droite passant par les pinces de fixation).

Les deux éléments de repérage horizontal 70, 80 sont également reliés entre eux par un élément de repérage médian 190 qui est associé mécaniquement aux deux éléments de repérage horizontaux 70, 80 de manière à rester constamment dans une position fixe par rapport à un plan vertical médian de symétrie de ces deux éléments 70, 80 sensiblement confondu avec un plan de symétrie PS de la monture (lui-même sensiblement confondu avec le plan sagittal PSAG du porteur). Cet élément de repérage médian porte une figure géométrique connue dont l'image, vue en projection dans le plan de capture d'image PCI par les moyens de capture d'image 90, permet, en combinaison avec les images des éléments de repérage 70, 80, de repérer dans l'espace l'orientation et la position du système de repérage 20, comme expliqué plus en détail dans la suite.

En l'espèce, cet élément de repérage médian est constitué par une barre de maintien 190 qui présente une direction longitudinale sensiblement perpendiculaire au plan de symétrie PS et donc au plan sagittal PSAG. Deux lumières 191, 192 de forme oblongue sont pratiquées dans la barre de maintien 190 en étant orientées selon la direction longitudinale de la barre. Les lumières 191, 192 accueillent deux plots de guidage 195, 196 attachés à la face supérieure des éléments de repérage 70, 80. Chaque moyen de repérage 70, 80 peut alors glisser par rapport à la barre de maintien 190 selon la direction longitudinale de la barre, les plots 195, 196 guidant le déplacement des moyens de repérage 70, 80 le long des lumières.

Cette mobilité de glissement des moyens de repérage 70, 80 par rapport à la barre de maintien 190 combinée à leur mobilité de pivotement autour de l'axe de pivotement A1 permet aux éléments de repérage horizontal 70, 80 d'être fixés sans contrainte sur les cercles 11, 12 par l'intermédiaire des baguettes 23, 24, de manière à suivre librement la composante horizontale de l'orientation des cercles 11, 12 de la monture 10.

La barre de maintien 190 comporte également sur sa tranche qui fait face aux moyens de capture d'image 90 une figure géométrique 193 constituée de bandes sombres espacées entre elles d'une distance connue. Comme expliqué plus loin, ces bandes sombres peuvent servir à calculer la distance séparant le système de repérage 20 des moyens de capture d'image 90 et ainsi déterminer le facteur d'échelle de l'image capturée.

Il est également prévu un moyen de centrage permettant de centrer le système de repérage 20 sur le plan de symétrie PS de la monture de telle sorte que le pontet nasal 15 soit centré sur l'axe A1.

Le système de repérage 20 comporte également un élément de repérage vertical 60 qui est également constitué par une plaque triangulaire d'une épaisseur donnée s'étendant dans un plan sensiblement perpendiculaire au plan moyen des deux éléments de repérage horizontal 70, 80 associés aux lentilles 100, 101. Cet élément de repérage 60 présente sur l'une de ses tranches qui est destinée à être orientée vers les moyens de capture d'image 90, une figure géométrique 61 constituée de motifs géométriques qui comme précédemment sont des bandes sombres séparées les unes des autres d'une distance connue et qui s'étendent selon la direction longitudinale de la tranche correspondante de l'élément de repérage 60. Il en résulte qu'ici, chaque bande sombre est disposée sensiblement à l'horizontale dans la configuration du porté et que la direction longitudinale de la figure géométrique 61 est sensiblement verticale.

L'élément de repérage vertical 60 est fixé sur la face supérieure de la barre de maintien 190 en son centre. La tranche de l'élément 60 portant la figure géométrique 61 s'étend dans un plan qui est sensiblement parallèle à la ligne joignant les centres de rotation CROG, CROD des yeux et qui forme, dans le plan sagittal PSAG, un angle fixe GAMMA de 30 degrés avec la normale N90 au plan de la face supérieure de la barre de maintien 190 (figure 2).

À proximité des extrémités libres des baguettes 23, 24, il est prévu deux montants 21, 22 parallèles entre eux et perpendiculaires aux baguettes 23, 24. Dans la configuration du porté, les montants 21, 22 sont sensiblement verticaux. Lorsque le système de repérage 20 est fixé sur la monture, les montants 21, 22 sont situés du côté des tempes droite et gauche du porteur, à proximité des branches 13, 14 de la monture 10 (voir figure 3).

Deux tiges 37, 38 horizontales sont montées coulissantes le long des montants 21, 22. Chaque tige comporte à son extrémité dirigée vers l'autre tige, une bille d'appui 30, 40. La structure du système de repérage 20 est conçue de telle sorte que les billes d'appui 30, 40 viennent en appui par gravitation contre les lentilles de présentation 100, 101, lorsque le système de repérage 20 est monté sur la monture 10 disposée sur le nez du porteur. Cet appui par gravitation est obtenu en concevant le système de repérage de telle sorte que son centre de gravité soit situé vers l'avant, c'est-à-dire du côté des figures géométriques. On peut également lester l'avant des plaques triangulaires 70, 80. En variante, pour appuyer les billes 30, 40 sur chaque lentille, on peut prévoir des moyens de rappel élastiques des tiges 21, 22 vers l'arrière. Le rappel des billes 30, 40 en appui contre la lentille de présentation correspondante 100, 101 permet de matérialiser un plan général de cette lentille.

Les moyens de capture d'image 90 consistent typiquement en un appareil de photographie numérique portatif ou monté sur support ou piètement.

Préférentiellement, les moyens de capture d'image 90 comporte une diode luminescente 92, ce qui permet, d'une part, d'obtenir un reflet cornéen aisément repérable sur l'image capturé, et, d'autre part, d'attirer le regard du porteur vers cette diode dont la position est connue. Le traitement de l'image capturée est alors facilité.

Le système de traitement et de calcul 93 de l'image acquise comporte ici un microordinateur sur lequel est installé un logiciel de traitement et de calcul de l'image acquise. En variante, on peut prévoir que le système de traitement et de calcul soit un système autonome qui comporte, d'une part, un écran d'affichage pour communiquer les résultats obtenus et, d'autre part, une connectique pour permettre de communiquer ces résultats à d'autres appareils. On peut également prévoir dans le cas d'un système autonome de traitement que ce système soit intégré ou non aux moyens de capture d'image 90.

Procédé

Le dispositif de détermination décrit ci-dessus permet de mettre en oeuvre le procédé suivant de détermination de la configuration du référentiel de chaque lentille correctrice à monter dans la monture, par rapport au référentiel du porteur.

Comme représenté sur la figure 4, l'opticien positionne la paire de lunettes de présentation surmontée du système de repérage 20 sur le nez du porteur. Le porteur est en configuration assise ou debout et sa tête est droite, c'est-à-dire que le plan de Francfort PF est sensiblement horizontal.

Comme représenté à la figure 3, les deux pinces de fixation 25, 26 de la baguette 23 sont appliquées sur la partie supérieure du cercle droit 11 de la monture 10. De même, les deux pinces de fixation 27, 28 de la baguette 24 sont appliquées sur la partie supérieure du cercle gauche 12 de la monture 10. Préférentiellement, les pinces de fixation 25, 26 et 27, 28 de chaque paire sont écartées le plus possible l'une de l'autre, de manière que la baguette 23, 24 correspondante suive la composante horizontale de l'orientation du cercle 11, 12 sur lequel elle est fixée. La composante horizontale de l'orientation de chaque cercle correspond globalement à l'inclinaison de la lentille de présentation associée par rapport au plan sagittal, en projection dans le plan de Francfort.

La barre de maintien 190 permet de s'assurer que les deux éléments de repérage horizontal 70, 80 restent sensiblement coplanaires. Il en résulte que l'élément de repérage vertical 60 s'étend bien dans le plan de symétrie de la monture PS (et donc dans le plan sagittal PSAG) lorsque le système de repérage 20 est monté sur la monture 10 (voir figures 3 et 5).

Chaque bille d'appui 30, 40 portée par la tige 37, 38 réglable en hauteur est disposée par l'opticien sensiblement à la hauteur des pupilles PG, PD des yeux. Le système de repérage 20 est conçu de telle sorte que les billes 30, 40 prennent appui par gravité contre les faces avant des lentilles de présentation 100, 101. L'appui par gravité des billes 30, 40 contre la lentille de présentation 100, 101 correspondante est réalisé à la faveur d'un basculement des baguettes 23, 24 autour d'un axe de basculement sensiblement parallèle à l'axe passant par les centres des deux pupilles (donc sensiblement perpendiculaire au plan sagittal PSAG et parallèle à l'axe X défini plus loin). Les pinces de fixation jouent ainsi un rôle de charnière pour le basculement du système de repérage autour de l'axe de basculement.

Il en résulte que la normale N90 au plan de la face supérieure de la barre de maintien 190 suit la composante verticale de l'orientation de la monture 10, qui correspond globalement à l'angle d'inclinaison, dans le pian sagittal PSAG, du plan moyen des cercles de la monture par rapport au plan vertical oeil PVO (figure 5).

Les deux points d'appui des pinces de fixation 25, 26 sur le cercle 11, la monture 10 et le point d'appui de la bille 30 sur la lentille de présentation 100 (c'est-à-dire le point de l'emplacement de la croix de montage) définissent un plan moyen PLD de la lentille de présentation 100 que l'on associe au plan moyen de la lentille correctrice dans la configuration du porté (figures 1 et 3). On définit de même le plan PLG de la lentille de présentation 101 passant par les deux points d'appui des pinces de fixation 27, 28 sur le cercle 12, la monture 10 et le point d'appui de la bille d'appui 40 sur la lentille de présentation 101.

Comme représenté sur la figure 1, on définit un référentiel associé à la monture (et donc indirectement à la tête du porteur) ayant un repère orthonormé (O,X,Y,Z) et matérialisé par le système de repérage 20. Le centre O du repère de ce référentiel est le milieu du segment joignant les pinces de fixation 26, 27. L'axe X est horizontal et passe par les pinces 26, 27. L'axe Y est perpendiculaire au plan de Francfort, donc ici vertical. Le plan OYZ est alors vertical et correspond au plan sagittal PSAG ainsi qu'au plan de symétrie PS. L'axe OZ est parallèle à la médiatrice AO des yeux. Le plan OXZ est parallèle au plan de Francfort PF et est donc ici horizontal. Le plan OXY est appelé plan vertical monture PVM et est sensiblement parallèle au plan facial de capture d'image PCI.

Détermination de l'orientation de chaque lentille par rapport à l'oeil correspondant du porteur

L'orientation de chaque lentille est donnée par les composantes, dans le référentiel (O,X,Y,Z) du vecteur normal au plan tangent à la lentille au point de la croix de montage CMG, CMD. Cette croix de montage correspond à un point de la lentille qui doit être situé en vis-à-vis de la pupille de l'oeil du porteur pour que la lentille exerce précisément ses fonctions de correction optique pour lesquelles elle a été conçue. La composante verticale de l'orientation de la lentille correspond à l'angle que forme l'axe, ou le vecteur, normal au plan de la lentille par rapport au plan facial, en projection dans le plan sagittal. On définit également la composante horizontale de l'orientation de la lentille qui correspond à l'angle que forme l'axe, ou le vecteur, normal au plan de la lentille par rapport au plan facial, en projection dans le plan de Francfort.

On cherche à déterminer l'orientation des plans PLG, PLD pour connaître l'orientation de chaque lentille correctrice à réaliser, par rapport au référentiel du porteur. Pour cela, on détermine l'orientation de l'axe XLG, XLD qui passe par le point d'appui des billes 30, 40 sur la lentille de présentation 100, 101 et qui est normal au plan PLG, PLD.

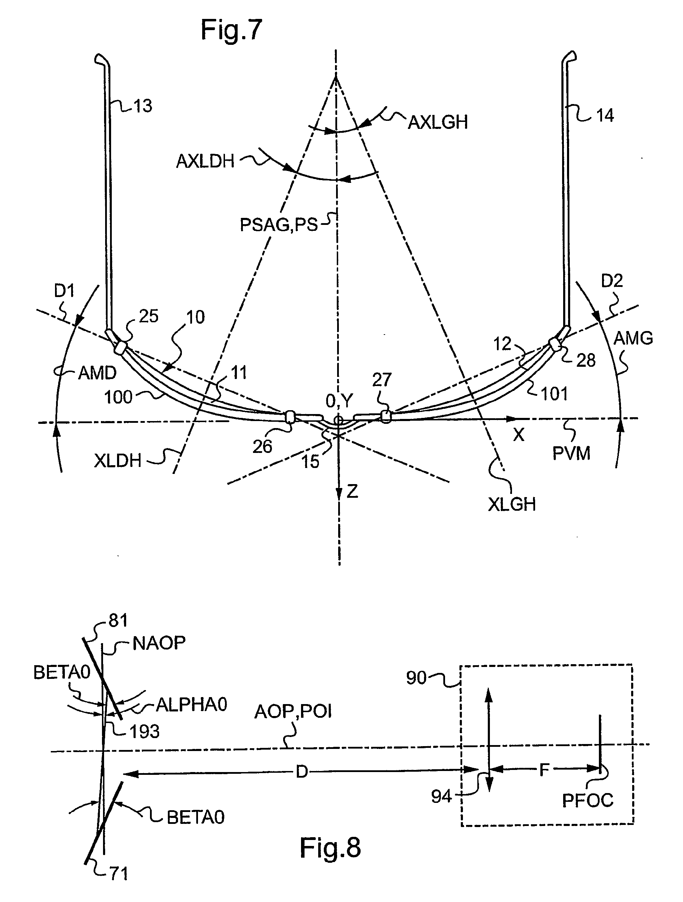

Comme représenté sur la figure 7, on définit les axes XLGH, XLDH comme les projections dans le plan horizontal, ou plan de Francfort, des axes XLG, XLD. De même, on définit les axes XLV comme les projections dans le plan sagittal des axes XLG, XLD (figure 5). Ici on considère que les projections dans le plan sagittal des axes XLG, XLD donnent chacune le même axe projeté XLV. En variante, il est possible de distinguer les deux projections des axes XLG, XLD dans le plan sagittal.

La composante horizontale de l'orientation de chaque lentille 100, 101 correspond ainsi à l'angle AXLGH, AXLDH que forme l'axe XLGH, XLDH avec le plan sagittal PSAG de la tête du porteur. De même la composante verticale de l'orientation de chaque lentille 100, 101 correspond à l'angle AXV que forme l'axe XLV avec le plan de Francfort. Reste alors à déterminer les angles AXLGH, AXLDH et AXV pour déterminer l'orientation de chaque lentille par rapport au porteur.

L'angle AXLDH, formé entre l'axe XLDH et sa projection sur le plan sagittal PSAG, correspond sensiblement à l'angle AMD formé dans le plan horizontal, ou plan de Francfort PF, entre, d'une part, la droite D1 passant par les pinces de fixation 25, 26 situées sur le cercle droit 11, à proximité respectivement de la branche droite 13 et du pontet nasal 15 et, d'autre part, le plan vertical monture PVM. De même, l'angle AXLGH correspond sensiblement à l'angle AMG formé entre, d'une part, la droite D2 passant par les pinces de fixation 27, 28 situées sur le cercle gauche 12 à proximité du pontet nasal 15 et de la branche gauche 14 et, d'autre part, le plan vertical monture PVM. Pour déterminer chacun des angles AXLGH, AXLDH, il suffit ainsi de déterminer les angles AMG et AMD.

Comme représenté sur la figure 5, l'angle AXV est sensiblement égal à l'angle AMV formé, en projection dans le plan sagittal PSAG, entre, d'une part, le plan vertical oeil PVO et, d'autre part, le plan moyen PMC des deux lentilles 100, 101 (ou encore des deux cercles 11, 12 de la monture 10). Pour déterminer l'angle AXV, il suffit ainsi de déterminer l'angle AMV.

L'opticien positionne l'appareil de capture d'images portatif 90 en regard de la tête du porteur et capture dans le plan de capture d'image PCI l'image de la tête du porteur équipé de la paire de lunettes de présentation surmontée du système de repérage 20. L'image obtenue correspond à l'image de la figure 3. La capture de l'image est réalisée typiquement à une distance du porteur comprise entre 50 et 120 centimètres dans un plan de capture d'image PCI. Ce plan de capture d'image PCI est facial, c'est-à-dire sensiblement parallèle aux plans PVO et PVM (figures 4 et 5).

Comme représenté sur la figure 2, on définit l'angle ARHD comme étant l'angle formé, dans le plan horizontal ou plan de Francfort PF, entre, d'une part, le plan vertical monture PVM, et d'autre part, la direction longitudinale de la figure géométrique 71. Lorsque cet angle ARHD varie, l'écart entre les bandes noires varie lui aussi en projection dans le plan de capture d'image PCI. Cet angle ARHD est égal à la somme de l'angle AMD et de l'angle fixe TETA de 30 degrés. L'angle ARHD varie donc de la même manière que l'angle AMD. Il en est de même pour l'élément de repérage horizontal 80 pour lequel on définit l'angle ARHG comme la somme de l'angle AMG et de l'angle fixe TETA de 30 degrés.

Le système de traitement et de calcul 93 mesure l'écart entre les bandes sombres de la figure géométrique 71 de l'élément de repérage horizontal 70 sur l'image qu'il a capturée, dans la configuration du porté. Pour limiter les erreurs de mesure sur l'image capturée, dues aux pixels de l'image capturée, le système de traitement et de calcul 93 mesure l'écart entre les bandes deux à deux et calcule une moyenne de cet écart. Puis par comparaison avec une configuration de référence de la figure géométrique 71 pour laquelle l'angle ARHD et l'écart entre les bandes sont connus, il détermine la variation d'écart entre les bandes entre la configuration du porté et la configuration de référence. Puis, le système de traitement et de calcul 93 détermine, en fonction de cette variation d'écart, l'angle ARHD. L'angle AMD est ensuite déterminé à partir de l'angle ARHD.

Pour réaliser une comparaison valable des écarts entre les bandes, le système de traitement et de calcul doit prendre en compte le facteur d'échelle de l'image capturée. La connaissance du facteur d'échelle permet de ramener les valeurs d'écart de bandes mesurées sur l'image capturée et les valeurs d'écart de bandes de référence à la même échelle pour pouvoir réaliser une comparaison des écarts de bande. Ce facteur d'échelle est déterminé à partir de la distance qui sépare le système de repérage des moyens de capture d'image 90.

La distance de séparation, référencée D, peut être obtenue par la méthode de calcul exposée ci-dessous.

Comme schématisé sur la figure 8, la direction longitudinale de la figure géométrique 193 de la barre de maintien 190 forme un angle ALPHA0 avec la normale NAOP à l'axe optique AOP, en vue de dessus du dispositif de détermination. De même la direction longitudinale de chaque figure géométrique 71, 81 des éléments de repérage correspondant 70, 80 forme un angle BETAO avec la direction longitudinale de la figure géométrique 193 de la barre de maintien 190. On considère également que les figures géométriques 71, 81 ont chacune la même longueur H connue et que la figure géométrique 193 possède également une longueur L connue.

On mesure, en prenant l'écart entre les bandes sombres, la longueur apparente T de la figure géométrique 193 de la barre de maintien 190 dans le plan focal PFOC de l'objectif 94. On a la relation :

$$L*\cos(ALPHA0)*F/D = T$$

F étant la distance focale de l'objectif 94 et D la distance entre l'appareil de capture d'image 90 et l'origine O du repère associé au système de repérage 20.

On mesure également les longueurs apparentes T1 et T2 des figures géométriques 71, 81 dans le plan focal PFOC. On obtient les relations :

$$H*\cos(BETA0-ALPHA0)*F/D = T1 \text{ et } H*\cos(BETA0 + ALPHA0)*F/D = T2.$$

On calcule alors de façon approchée BETAO en sommant les deux longueurs apparentes T1 et T2 :

$$T1+T2 = 2*\cos BETA0 *\cos ALPHA0 * H*F/D$$

et,
en considérant cos ALPHA0 proche de 1, on obtient :

$$T1+T2 = 2* \cos BETA0 * H * T / L$$

On en tire une valeur approchée de BETAO.

On calcule ensuite le rapport K de ces deux longueurs pour éliminer H*F/D : K= (cos BETAO cos ALPHA0 + sin BETAO sin ALPHA0) / (cos BETAO cos ALPHA0 - sin BETAO sin ALPHA0). Les valeurs de K et BETAO étant connues, on calcule ALPHA0 avec la relation :

$$\tan(ALPHA0) = [(K-1)*\cos BETA0] / [(K+1)*\sin BETA0]$$

On en déduit donc la distance D, grâce à la mesure de T, les valeurs de F et L étant connues :

$$D = L*\cos(ALPHA0)*F/T$$

Il est également possible d'utiliser un télémètre à diodes laser pour déterminer directement cette distance de séparation.

Le système de traitement et de calcul 93 mesure également l'écart entre les bandes sombres de la figure géométrique 81 de l'élément de repérage horizontal 80 sur l'image qu'il a capturée, dans la configuration du porté. Comme précédemment, pour limiter les erreurs de mesure sur l'image capturée, dues aux pixels de l'image capturée, le système de traitement et de calcul 93 mesure l'écart entre les bandes deux à deux et calcule une moyenne de cet écart. Puis, par comparaison avec une configuration de référence de la figure géométrique 81 pour laquelle l'angle ARHG et l'écart entre les bandes est connu, il détermine la variation d'écart entre les bandes entre la configuration du porté et la configuration de référence. La comparaison des écarts de bandes est réalisée en tenant compte du facteur d'échelle de l'image capturée. Puis, le système de traitement et de calcul 93 détermine, en fonction de cette variation d'écart, l'angle ARHG. Puis l'angle AMG est déterminé à partir de l'angle ARHG.

Comme représenté sur la figure 2, on définit l'angle ARV comme étant l'angle formé, en projection dans le plan sagittal PSAG, entre, d'une part, le plan vertical monture PVM, et d'autre part, la direction longitudinale de la figure géométrique 61. Lorsque cet angle ARV varie, l'écart entre les bandes noires varie lui aussi en projection dans le plan de capture d'image PCI. Cet angle ARV est égal à la somme de l'angle AMV et de l'angle fixe GAMMA de 30 degrés que forme la figure géométrique 61 avec la normale N90. L'angle ARV varie donc de la même manière que l'angle AMV.

Le système de traitement et de calcul 93 mesure alors l'écart entre les bandes de la figure géométrique 61 sur l'image qu'il a capturée. Comme précédemment, il est prévu une configuration de référence de la figure géométrique 61 pour laquelle le couple de données constituées de l'angle ARV et de l'écart entre les bandes est connu. Par comparaison des valeurs de mesures d'écart de bandes réalisées sur l'image capturée avec les valeurs d'écart de bandes de référence, le système de traitement et de calcul 93 en déduit une variation d'écart. Comme précédemment, la comparaison des écarts de bandes est réalisée en tenant compte du facteur d'échelle de l'image capturée. Puis, le système de traitement et de calcul détermine, en fonction de cette variation d'écart, l'angle ARV. Puis l'angle AMV est déterminé à partir de l'angle ARV.

Le système de traitement et de calcul détermine ainsi l'orientation des axes XLG, XLD de chaque lentille gauche et droite par rapport au plan de Francfort et au plan sagittal PSAG. En conséquence, l'orientation des plans associés à chaque lentille de présentation gauche et droite est connue dans le référentiel du porteur. On connaît alors l'orientation de chaque lentille correctrice à monter dans la monture par rapport à l'oeil correspondant.

Dans une configuration du porteur telle que le porteur regarde droit devant lui face à l'appareil de capture d'image 90, c'est-à-dire telle que le plan sagittal PSAG est perpendiculaire au plan de capture d'image PCI, l'angle AMG doit être égal à l'angle AMD.

Lorsque les angles AMG, AMD présentent un écart de valeurs, on en déduit alors que la tête du porteur est tournée d'un angle de posture APIV défini comme suit.

Comme représenté sur la figure 6, l'angle de posture APIV est l'angle formé, dans le plan horizontal, ou plan de Francfort PF, entre le plan sagittal PSAG et le plan d'observation POI qui contient le centre 96 de la pupille 95 de l'appareil de capture d'image 90 et l'axe de rotation vertical de la tête ART.

La différence de valeur entre l'angle AMG et l'angle AMD est proportionnelle à la valeur de l'angle de posture APIV. Le système de traitement et de calcul 93 calcule alors une valeur de l'angle de posture APIV en fonction de la différence de valeur mesurée entre les angles AMG, AMD. Cette valeur de l'angle de posture APIV permet de corriger les valeurs des angles AMG, AMD.

En outre, pour améliorer la personnalisation de la conception optique de chaque lentille, on détermine également des paramètres géométrico-morphologiques qui permettent d'améliorer la répartition des gradients d'indice lors de la conception de la lentille. Le système de traitement et de calcul 93 détermine ainsi par reconnaissance d'image, en tenant compte du facteur d'échelle, les cotes de largeur B et de longueur A de chacun des cercles 11, 12 entourant les lentilles de présentation.

Le système de traitement et de calcul 93 détermine également par reconnaissance d'image la hauteur HG, HD de l'oeil OG, OD correspondant en mesurant sur l'image capturée, en tenant compte du facteur d'échelle, la distance séparant la ligne de séparation 34 positionnée sur le centre de la pupille PG, PD et le point de référence pris comme étant le point le plus bas de la lentille.

Pour mesurer l'écart interpupillaire PDS, le système de traitement et de calcul 93 détermine par reconnaissance d'image, pour chaque oeil, le centre de la pupille (ou de l'iris). Le segment défini par les centres ainsi obtenus des deux pupilles PG, PD fournit la distance interpupillaire PDS. On peut également mesurer les demi-écarts pupillaires PDSD, PDSG en mesurant la position horizontale du centre de chaque pupille PG, PD par rapport au centre du pontet nasal 15.

La mesure de l'écart interpupillaire PDS ou ses demi-écarts pupillaires PDSD, PDSG est réalisée ici pour une configuration de convergence de référence. Selon cette configuration de convergence de référence, il est prévu que les yeux du porteur fixent une lumière sur les moyens de capture d'images, par exemple la diode luminescente 92, que le porteur vise du regard. Le système de traitement et de calcul détermine alors, en fonction de la distance de prise de capture et de la valeur d'écart interpupillaire PDS (ou de demi-écart) correspondante mesurée, la valeur de cet écart interpupillaire pour une vision à l'infini.

Bien entendu, toutes les mesures réalisées sur l'image tiennent compte du facteur d'échelle.

Il est également possible d'améliorer la précision de calcul des valeurs de la distance interpupillaire, demi-écart interpupillaire, et de la cote de largeur A en corrigeant ces valeurs calculées en fonction des angles AMD, AMG précédemment calculés, ou encore de l'angle de posture APIV ou de la différence de distance entre les yeux OD, OG et les lentilles 100, 101 correspondantes. De même, les mesures de la cote de hauteur B et de la hauteur HG, HD de la pupille de chaque oeil peuvent être améliorées en tenant compte de la valeur de l'angle AMV.

L'inclinaison des directions longitudinales des figures géométriques 71, 81 par rapport aux directions longitudinales des baguettes 23, 24 est utilisée pour augmenter les valeurs de variations d'écart entre les bandes lorsque les angles AMG, AMD varient. L'écart entre deux bandes résultant de la variation des angles AMG, AMD par rapport aux configurations de référence connues est ainsi plus facilement repérable. Ainsi la comparaison des écarts est représentative de la composante horizontale de l'orientation de chacun des cercles 11, 12 de la monture, et donc de chaque lentille, ce qui limite les erreurs de mesure.

Il en est de même de l'inclinaison de la direction longitudinale de la figure géométrique 61 par rapport à la normale N90. En effet, cette inclinaison augmente les valeurs de variations d'écart entre les bandes lorsque l'angle AMV varie.

Détermination de la distance entre la lentille et le centre de l'oeil correspondant du porteur

On souhaite ensuite déterminer la distance, suivant une direction horizontale du plan sagittal PSAG, c'est-à-dire suivant l'axe Z, entre la lentille 100 et le centre CROD de l'oeil droit OD, ainsi que la distance suivant cette même direction entre la lentille 101 et le centre CROG de l'oeil gauche OG. On applique à cet effet le procédé objet de la présente invention, pour déterminer la position suivant l'axe Z du centre de rotation CROG, CROD en tant que point remarquable de l'oeil dans le référentiel (O,X,Y,Z) lié à la tête du porteur et matérialisé par le système de repérage 20. On en déduit ensuite, compte-tenu de la configuration désormais connue de la lentille concernée dans le référentiel (O,X,Y,Z), la distance recherchée entre le centre de rotation de l'oeil et la lentille.

Au cours d'une première étape S1, on dispose l'appareil de capture d'image 90 en regard du visage du porteur, de telle sorte que la tête du porteur présente une première posture relative par rapport à la pupille d'entrée 95 de l'appareil de capture d'image 90.

Cette première posture relative est telle que l'appareil de capture d'image observe le porteur globalement de face et non pas de profil. Le fait de mesurer la position des yeux à partir d'une prise d'image de face offre plusieurs avantage, par comparaison à une prise d'image de profil. L'opération de traitement d'image est en effet, en pratique, plus fiable dans la mesure où les cercles et branches de la monture ne risquent pas de masquer l'oeil et où, grâce à la méthode de calcul que nous examinerons plus loin, la reconnaissance du point de la face arrière de la lentille en regard de l'oeil (qui était périlleuse) n'est alors plus nécessaire. En outre, une ou plusieurs prises de vue de face du porteur permettent de mesurer d'autres paramètres géométrico-morphologiques du porteur, tels que les écarts interpupillaires et hauteurs des yeux, le galbe de la monture, l'angle pantoscopique de chaque lentille au porté (angle que forme le plan général de la lentille par rapport à la verticale), ainsi que nous l'avons vu précédemment, ou encore le comportement visuel du porteur (en particulier sa tendance à bouger plus ou moins les yeux ou la tête lorsqu'il scrute le champ visuel).

Dans la suite, les occurrences correspondant à cette première posture des différents éléments de géométrie définis, tels que les droites, les plans et les angles, sont affectés de l'indice 1. Il en est ainsi en particulier du plan d'observation POI dont cette première occurrence est notée POI1, du plan sagittal PSAG dont cette première occurrence est notée PSAG1, de l'angle de posture APIV dont cette première occurrence est notée APIV1, du repère (O,X,Y,Z) dont cette première occurrence est notée (O1,X1,Y1,Z1).

Au cours d'une seconde étape S2, on capture dans cette première posture relative faciale, au moyen de l'appareil de capture d'image 90 et comme indiqué précédemment, une première image plane de la tête du porteur.

Au cours d'une troisième étape S3, le système de traitement et de calcul 93 identifie, sur cette première image, l'image d'un premier point de référence prédéterminé de chaque oeil.

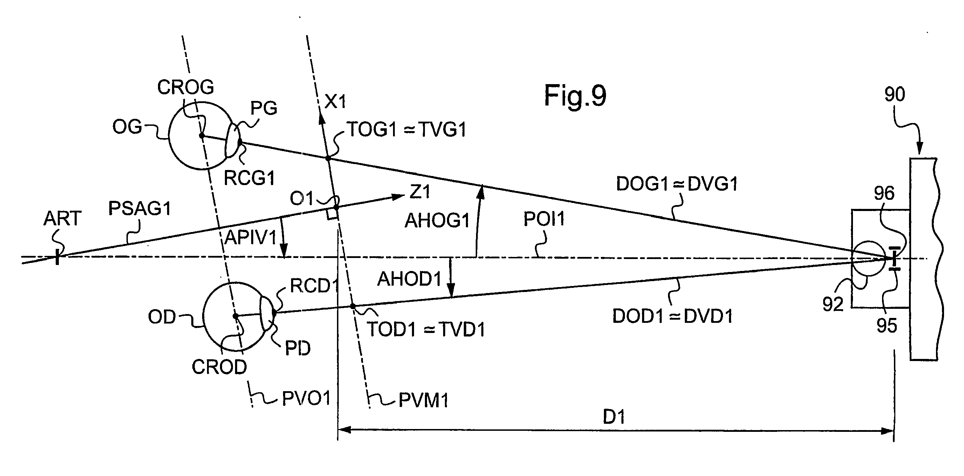

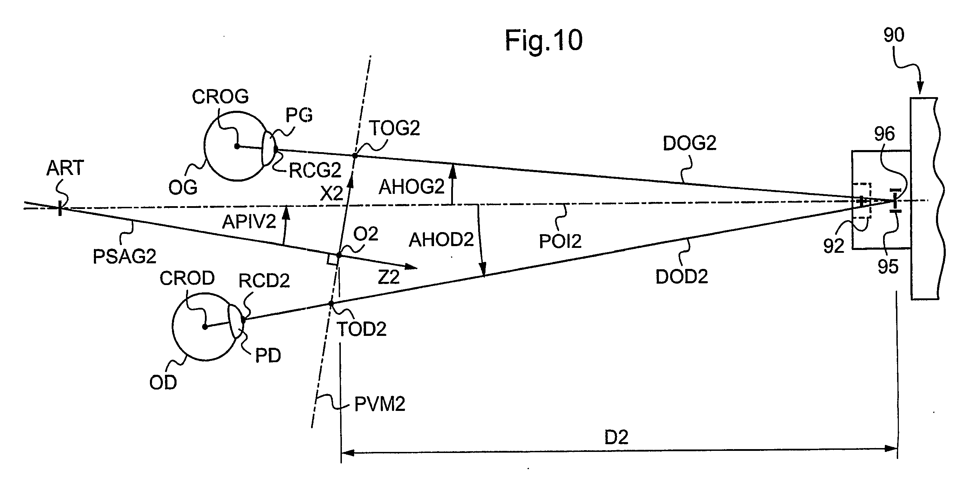

Ce premier point de référence de l'oeil est, dans l'exemple illustré par les figures 9 et 10, le reflet RCG1, RCD1 sur la cornée de l'oeil gauche OG et de l'oeil droit OD respectivement, de la source lumineuse visible que constitue la diode 92.

Le système de traitement et de calcul 93 calcule aussi la configuration de l'appareil de capture d'image 90 dans la première occurrence (O1,X1,Y1,Z du référentiel (O,X,Y,Z) lié à la tête du porteur, à partir de la première image capturée du système de repérage 20, dans la première posture. Il calcule en particulier une première valeur APIV1 de l'angle de posture APIV et la position du centre 96 de la pupille d'entrée 95 de l'appareil de capture d'image 90. Typiquement, comme indiqué précédemment, le système de traitement et de calcul 93 calcule la première valeur APIV1 de l'angle de posture APIV en fonction de la différence entre les valeurs mesurées AMG1, AMD1 des angles AMG, AMD dans cette première posture, tandis que la distance entre l'origine O1 du repère et le centre 96 de la pupille 95 est obtenue par un calcul de mise à l'échelle déduite de la grandeur apparente d'une figure géométrique du système de repérage 20 dont la taille réelle est connue.

Au cours d'une quatrième étape S4, on modifie l'angle de prise de vue : on dispose l'appareil de capture d'image 90 en regard du visage du porteur, de telle sorte que la tête du porteur présente une seconde posture relative, différente de la première, par rapport à la pupille d'entrée 95 de l'appareil de capture d'image 90. On déplace à cet effet, soit la position absolue de la tête du porteur, par exemple en demandant ou en incitant celui-ci à faire pivoter sa tête autour de l'axe de rotation vertical ART de celle-ci, soit en déplaçant l'appareil de capture d'image 90 par rapport au porteur, soit encore en combinant ces deux déplacements.

En particulier, la position relative de la pupille de l'appareil de capture d'image et de l'axe de rotation vertical ART de la tête du porteur restant identique pendant les deux captures d'images, le porteur suit du regard une source lumineuse visible formant un point de visée en faisant pivoter sa tête autour dudit axe de rotation vertical ART.

On considère ici par exemple que les positions relatives de la pupille de l'appareil de capture d'image et de l'axe de rotation vertical ART de la tête du porteur sont identiques dans lesdites première et seconde postures, si, entre lesdites première et seconde postures, la tête du sujet ne se déplace pas transversalement de plus de 20 pourcent de la distance existant entre la pupille de l'appareil de capture d'image et l'axe de rotation vertical ART dans la première posture, dans la direction perpendiculaire à l'axe optique de l'appareil de capture d'image.

Par exemple, pour une distance de 1 mètre existant entre la pupille de l'appareil de capture d'image et l'axe de rotation vertical ART dans la première posture, un déplacement transversal de 20 pourcent de cette distance correspond à un déplacement transversal de 20 centimètres suivant une direction perpendiculaire à l'axe optique de l'appareil de capture d'image, d'un côté ou de l'autre de cet axe optique.

Le sujet tourne de préférence simplement la tête autour de l'axe de rotation vertical ART, en gardant la tête droite, c'est-à-dire un plan de Francfort horizontal. Cette rotation de la tête du sujet autour de l'axe de rotation vertical ART présente de préférence une amplitude comprise entre 5 et 60 degrés.

L'angle de posture APIV est ainsi modifié.

La seconde posture relative est, comme la première posture, telle que l'appareil de capture d'image observe le porteur globalement de face et non pas de profil.

Dans la suite, les occurrences correspondant à cette seconde posture des différents éléments de géométrie définis, tels que les droites, les plans et les angles, sont affectés de l'indice 2. Il en est ainsi en particulier du plan d'observation POI dont cette seconde occurrence est notée POI2, du plan sagittal PSAG dont cette seconde occurrence est notée PSAG2, de l'angle de posture APIV dont cette seconde occurrence est notée APIV2, du repère (O,X,Y,Z) dont cette seconde occurrence est notée (O2,X2,Y2,Z2).

Au cours d'une cinquième étape S5, on capture dans cette seconde posture relative, au moyen de l'appareil de capture d'image 90, une seconde image plane de chaque oeil.

Au cours d'une sixième étape S6, le système de traitement et de calcul 93 identifie, sur cette seconde image, l'image d'un second point de référence prédéterminé de chaque oeil. Ce second point de référence de l'oeil est, dans l'exemple illustré par les figures 9 et 10, le reflet RCG2, RCD2 sur la cornée de l'oeil gauche OG et de l'oeil droit OD respectivement, de la diode 92. Les premier et second points de référence de chaque oeil sont donc dans cet exemple confondus en un même point de l'oeil considéré qui est approximativement le centre de la cornée ou de l'iris.

Le système de traitement et de calcul 93 calcule la configuration de l'appareil de capture d'image 90 dans l'occurrence (O2,X2,Y2,Z2 du référentiel (O,X,Y,Z) lié à la tête du porteur, à partir de la seconde image capturée du système de repérage 20, comme indiqué précédemment. Il calcule en particulier une seconde valeur APIV2 de l'angle de posture APIV et la position du centre 96 de la pupille d'entrée 95 de l'appareil de capture d'image 90.

Au cours d'une septième étape S7, le système de traitement et de calcul 93 calcule, dans la première posture relative et pour chaque oeil OD, OG, respectivement une première droite d'observation DOD1, DOG1 reliant le centre de pupille 96 de l'appareil de capture d'image 90 et le premier point de référence de l'oeil RCG1, RCD1 et, dans la seconde posture relative, une seconde droite d'observation DOD2, DOG2 reliant le centre de pupille 96 de l'appareil de capture d'image 90 et le second point de référence de l'oeil RCG2, RCD2.

Au cours d'une huitième étape S8, le système de traitement et de calcul 93 effectue un calcul pour vérifier si les premières droites d'observation DOG1, DOD1 présentent, par rapport à la tête du porteur, des configurations respectives sensiblement distinctes de celles des secondes droites d'observation DOG2, DOD2. Dans la négative, le système de traitement et de calcul 93 émet un message d'erreur et on exécute à nouveau les étapes S1 à S3 ou les étapes S4 à S6. Dans l'affirmative, le système de traitement et de calcul 93 procède à l'étape suivante S9.

Par ailleurs, le caractère facial de la prise de vue, évoqué précédemment, peut être quantifié de la façon suivante. La première posture relative est telle que ces premières droites d'observation DOD1, DOG1 forment chacune avec leurs projections sur le plan sagittal PSAG1 et sur le plan de Francfort PF du porteur des angles respectifs inférieurs à 45 degrés. De même, la seconde posture relative est telle que ces secondes droites d'observation DOD2, DOG2 forment chacune avec leurs projections sur le plan sagittal PSAG2 et sur le plan de Francfort PF du sujet des angles respectifs inférieurs à 45 degrés.

La conformité à ce critère est vérifiée par le système de traitement et de calcul 93. A défaut de satisfaction du critère, le système de traitement et de calcul 93 émet un message d'erreur et on modifie la première posture et/ou la seconde posture relative avant de capturer une nouvelle première image. Si le critère est satisfait, on passe à l'étape suivante.

Au cours d'une neuvième étape S9, le système de traitement et de calcul 93 calcule la position du centre de rotation CROG, CROD de chaque oeil OG, OD en fonction des images des premier et second points de référence de l'oeil concerné et des première et seconde valeurs APIV1, APIV2 de l'angle de posture APIV.

Le système de traitement et de calcul 93 effectue ensuite un calcul de rapprochement des images des premier et second points de référence de l'oeil pour en déduire un déplacement angulaire apparent de l'oeil à partir des deux points de vue différents correspondant aux première et seconde postures relatives. Un calcul de parallaxe permet alors, compte-tenu des informations sur les deux points de vue que constituent les première et seconde valeurs APIV1, APIV2 de l'angle de posture APIV, d'obtenir les positions recherchées des centres CROG, CROD de l'oeil suivant l'axe Z.

Pour calculer la position du centre de rotation CROG, CROD de l'oeil, le système de traitement et de calcul 93 calcule, à partir de l'image du premier point de référence de l'oeil concerné et de la première valeur de l'angle de posture APIV1, les coordonnées, dans ledit référentiel (O,X,Y,Z) de la tête du porteur, de la première droite d'observation DOD1, DOG1 reliant le centre 96 de la pupille d'entrée 95 de l'appareil de capture d'image 90 et le premier point de référence de l'oeil OD, OG. De même, le système de traitement et de calcul 93 calcule, à partir de l'image du second point de référence de l'oeil et de la seconde valeur de l'angle de posture APIV2, les coordonnées, dans ledit référentiel (O,X,Y,Z) de la tête du porteur, de la seconde droite d'observation DOD2, DOG2 reliant le centre de pupille de l'appareil de capture d'image et le second point de référence de l'oeil.

Le système de traitement et de calcul 93 calcule alors la position du centre de rotation de chaque oeil CROG, CROD dans le référentiel (O,X,Y,Z) en fonction des coordonnées des premières et secondes droites d'observation DOG1, DOG1, DOD1, DOG2, DOD2. La position du centre de rotation d'oeil' gauche CROG est calculée en tant que position du point d'intersection ou, si ces droites ne sont pas rigoureusement sécantes, de plus grande proximité des deux droites d'observation DOG1, DOG2. De même, la position du centre de rotation d'oeil droit CROD est calculée en tant que position du point d'intersection ou, si ces droites ne sont pas rigoureusement sécantes, de plus grande proximité des deux droites d'observation DOD1, DOD2.

On prend en considération la direction de regard du porteur.

Lors des première et seconde captures d'images, l'oeil regarde la diode luminescente 92 qui attire son regard. Le centre de cette diode 92 constitue, dans chacune des première et seconde postures relatives, respectivement des premier et second points de visée. Les positions de ces points de visée sont donc connues dans le référentiel de l'appareil de capture d'image 90. Les points de visée sont en l'espèce situés dans le plan d'observation POI à proximité de la pupille de l'appareil de capture d'image.

Le calcul de la position du centre de rotation CROD, CROG de chaque oeil est alors de plus fonction des positions des points de visée que matérialise la diode 92 dans les deux postures.

On définit dans la première posture relative, deux premières droites de visée DVD1, DVG1 reliant respectivement les points de référence RCD1, RCG1 au point de visée, c'est-à-dire à la diode 92 dans la première posture. On définit de même, dans la seconde posture relative, deux premières droites de visée DVD2, DVG2 reliant respectivement les points de référence RCD2, RCG2 au point de visée, c'est-à-dire à la diode 92 dans la seconde posture.

Comme la diode 92 est, dans l'exemple illustré par les figures 9 et 10, située à proximité de la pupille d'entrée 95 de l'appareil de capture d'image 90, on peut ici considérer, pour le calcul des positions des centres CROG, CROD dans le référentiel (O,X,Y,Z), que les yeux OD, OG regardent sensiblement vers la pupille d'entrée de l'appareil de capture d'image 90 dans chacune des première et seconde postures. Il en résulte que, en première posture, les droites de visée DVG1, DVD1 sont respectivement confondues avec les droites d'observation DOG1, DOD1 et que, de même, en seconde posture, les droites de visée DVG2, DVD2 sont respectivement confondues avec les droites d'observation DOG2, DOD2. C'est par cette approximation que l'on prend en compte, dans cet exemple, la position du point de visée dans les première et seconde postures relatives.

On définit alors deux angles horizontaux d'observation gauche et droit AHOG, AHOD que forment respectivement les droites d'observation gauche et droite DOG, DOD avec leurs projections respectives sur le plan d'observation POI. Les occurrences de ces angles dans les première et seconde postures, respectivement notées AHOG1, AHOD1, AHOG2, AHOD2, sont calculées par le système de traitement et de calcul 93. A cet effet, le système de traitement et de calcul 93 calcule, pour chaque posture, la position du plan d'observation POI en fonction des première et seconde images capturées correspondantes du système de repérage 20 et détermine les angles apparents sous lesquels ont été vus les points de référence, c'est-à-dire en l'espèce les reflets cornéens RCG1, RCD1, RCG2, RCD2, par l'appareil de capture d'image 90 dans les première et seconde postures relatives. Si, comme cela sera le plus souvent le cas, le plan d'observation POI est approximativement confondu avec l'axe optique AOP de l'appareil de capture d'image 90, les angles recherchés AHOG et AHOD sont égaux aux angles apparents relevés. Dans le cas contraire, le correctif correspondant est appliqué aux angles apparents pour en déduire les angles recherchés AHOG et AHOD.

A partir des valeurs ainsi calculées de ces angles, le système de traitement et de calcul 93 calcule, pour chaque posture, les traces respectives TOG, TOD des droites d'observation DOG, DOD dans le plan vertical monture PVM. Ces traces sont bien entendu des points qui sont notés TOG1, TOD1 pour la première posture et TOG2, TOD2 pour la seconde posture.

On définit également les traces respectives TVG, TVD des droites de visée DVG, DVD dans le plan vertical monture PVM. Comme le porteur regarde en l'espèce en direction de la pupille de l'appareil de capture d'image, ces points TVG, TVD sont approximativement confondus avec les points TOG, TOD dans les première et seconde postures. Plus précisément, le système de traitement et de calcul 93 calcule les abscisses respectives X(TOG1) et X(TOD1) des points TOG1 et TOD1 suivant l'axe X dans la première occurrence (01,X1,Y1,Z1) du repère (O,X,Y,Z), ainsi que les abscisses respectives X(TOG2) et X(TOD2) des points TOG2 et TOD2 dans la seconde occurrence (O2,X2,Y2,Z2 du repère (O,X,Y,Z). Il peut alors en déduire les distances recherchées entre les centres de rotation CROG, CROD des yeux gauche et droit, d'une part, et le plan vertical monture PVM, d'autre part, qui correspondent à l'opposé des abscisses suivant l'axe Z, notées respectivement Z(CROG), Z(CROD), de ces centres CROG, CROD. On peut appliquer les formules suivantes :

$$Z(CROD) = - Abs\ (\ (X(TOD1) - X(TOD2))$$
$$/\ (tan(AHOD1 + APIV1) - tan(AHOD2 + APIV2))\ )$$

$$Z(CROG) = - Abs\ (\ (X(TOG1) - X(TOG2))$$
$$/\ (tan(AHOG1 + APIV1) - tan(AHOG2 + APIV2))\ )$$

où "Abs" est la fonction de valeur absolue et "tan" la fonction de tangente.

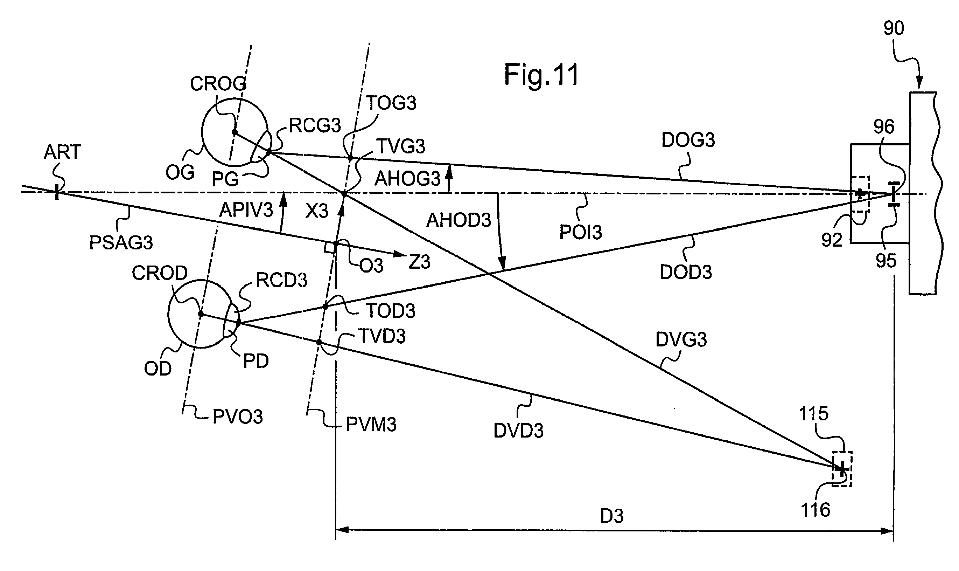

La figure 11 illustre une variante d'exécution de l'invention, qui peut être avantageuse pour un gain de précision ou pour réaliser des captures d'image pour des directions de regard correspondant aux visions de loin et de près, ou encore pour améliorer le calcul de rayon d'oeil.

Le dispositif est ici complété par une seconde source lumineuse visible 115 telle qu'une diode, dont le centre 116 constitue alors un point de visée attirant le regard du porteur. Le procédé reprend pour l'essentiel l'ensemble des étapes précédemment décrites. Seuls seront décrits les ajouts ou modifications opérés par rapport au mode de réalisation qui vient d'être décrit en détail.

Au cours d'une dixième étape S10, on prévoit une troisième posture relative (O3,X3,Y3,Z3) de la tête du sujet par rapport à la pupille d'entrée 95 de l'appareil de capture d'image 90. Cette troisième posture est identique ou distincte des première et seconde postures relatives.

La figure 11 illustre une troisième posture relative qui se substitue ou s'ajoute à la première ou seconde posture et dans laquelle une troisième image du visage du porteur et du système de repérage 20 est capturée.

Dans les première et seconde postures illustrées par les figures 9 et 10, les points de référence pris en compte pour le calcul de parallaxe sont les reflets cornéens de la diode 92 qui est située à proximité de la pupille d'entrée de l'appareil de capture d'image 90. Dans la troisième posture illustrée par la figure 11, on prévoit que le porteur regarde en direction de la diode 115, dont la position par rapport à la pupille 95 de l'appareil de capture d'image 90 est connue et entrée en paramètre dans la mémoire du système de traitement et de calcul 93. La position de la diode 115 est, par rapport au référentiel lié à la tête du sujet (O,X,Y,Z), distincte de celle de la diode 92.

Dans une onzième étape S11, l'appareil de capture d'image 90 capture dans cette troisième posture relative une troisième image plane de face de la tête du sujet, avec le système de repérage 20 et les yeux.

Le système de traitement et de calcul 93 calcule à partir de la troisième image capturée une troisième valeur APIV3 de l'angle de posture.

Dans une douzième étape S12, le système de traitement et de calcul 93 identifie, sur cette troisième image, l'image d'un troisième point de référence prédéterminé de chaque oeil. Ce point de référence est ici le reflet RCG3, RCD3 de la diode 115 respectivement par la cornée de l'oeil gauche et de l'oeil droit. Il en est ainsi en raison du fait que les yeux observent la diode 115.

Puis, au cours d'une treizième étape S13, le système de traitement et de calcul 93 calcule le rayon ROG, ROD de chaque oeil en fonction :

- des images des reflets cornéens RCG1, RCD1 de la première image et/ou des reflets cornéens RCG2, RCD2 de la seconde image, ainsi que de l'image des reflets cornéens RCG3, RCD3 de la troisième image,
- de la première et/ou seconde valeur APIV1, APIV2 et de la troisième valeur APIV3 du paramètre de posture APIV et

- des positions connues des points de visée 92, 116 par rapport à la pupille 95 de l'appareil de capture d'image 90.

Le groupe d'étapes S10, S11, S12 est en l'espèce distinct du groupe d'étapes S1, S2, S3 d'une part et du groupe d'étapes S4, S5, S6 d'autre part. En variante, on pourra prévoir que le groupe d'étapes S10, S11, S12 soit confondu avec l'un des groupes d'étapes S1, S2, S3 et S10, S11 S12. On réalise alors en combinaison, à partir de seulement deux images capturées de face, le calcul à la fois de la position de l'oeil par rapport à la lentille et de son rayon.

Le système de traitement et de calcul 93 calcule, à partir de l'image du troisième point de référence RCG3, RCD3 (reflet cornéen de la diode 115) de l'oeil concerné et de la troisième valeur de l'angle de posture APIV3, les coordonnées, dans le référentiel (O,X,Y,Z) de la tête du porteur, d'une troisième droite d'observation DOD3, DOG3 reliant le centre 96 de la pupille d'entrée 95 de l'appareil de capture d'image 90 et le troisième point de référence RCG3, RCD3 (reflet cornéen de la diode 115) de l'oeil OD, OG.

On prend en considération la direction de regard du porteur.

Lors des première et seconde captures d'images, chaque oeil regarde la diode luminescente 92. Mais dans la troisième configuration de la figure 11, chaque oeil regarde la diode luminescente 11 qui est distante de l'appareil de capture d'image. Le centre 116 de cette diode 115 constitue un troisième point de visée dont la position est connue dans le référentiel de l'appareil de capture d'image 90. Le calcul de la position du centre de rotation CROD, CROG de chaque oeil est alors de plus fonction des positions des points de visée que matérialise la diode 92 dans les deux postures.

On définit dans la troisième posture relative, deux troisièmes droites de visée DVD3, DVG3 reliant respectivement les troisièmes points de référence que constituent les reflets cornéens RCD3, RCG3 au point de visée, c'est-à-dire au centre 116 de la diode 115.

Comme la diode 115 située à distance de la pupille d'entrée 95 de l'appareil de capture d'image 90, les troisièmes droites de visée DVG3, DVD3 sont clairement distinctes des troisièmes droites d'observation DOG3, DOD3. Il faut donc spécifiquement prendre en compte, dans le calcul de position des centre de rotation des yeux, la position du troisième point de visée que constitue la diode 115.

La troisième occurrence AHOG3, AHOD3 de chacun des angles AHOG, AHOD (précédemment définis) dans la troisième posture est calculée par le système de traitement et de calcul 93, comme indiqué précédemment pour les première et seconde postures.

A partir des valeurs de ces angles, le système de traitement et de calcul 93 calcule les traces respectives TOG3, TOD3 des troisièmes occurrences des droites d'observation DOG3, DOD3 dans la troisième occurrence du plan vertical monture PVM3, ainsi que les traces respectives TVG3, TVD3 des troisièmes occurrences des droites de visée DVG3, DVD3 dans la troisième occurrence du plan vertical monture PVM3. Plus précisément, le système de traitement et de calcul 93 calcule les abscisses respectives X(TOG3) et X(TOD3) des points TOG3 et TOD3 suivant l'axe X dans la troisième occurrence (O3,X3,Y3,Z3) du repère (O,X,Y,Z), ainsi que les abscisses respectives X(TVG3) et X(TVD3) des points TVG3 et TVD3 dans la troisième occurrence (O3,X3,Y3,Z3 du repère (O,X,Y,Z). Il peut alors en déduire les distances recherchées entre les centres de rotation CROG, CROD des yeux gauche et droit, d'une part, et le plan vertical monture PVM, d'autre part, qui correspondent à l'opposé des abscisses suivant l'axe Z, notées respectivement Z(CROG), Z(CROD), de ces centres CROG, CROD.

Le système de traitement et de calcul 93 calcule de plus le rayon de l'oeil en tirant parti du fait qu'entre les première et seconde postures d'une part et la troisième posture d'autre part, la position angulaire de chaque oeil autour de son centre de rotation CROG, CROD a changé par rapport à la pupille d'entrée 95 de l'appareil de capture d'image 90.

Le système de traitement et de calcul 93 calcule le rayon de chaque oeil OD, OG à partir :

- des abscisses respectives X(TVG3), X(TVD3), X(TOG3), X(TOD3) des points TVG3, TVD3; TOG3, TOD3 dans la troisième occurrence (O3,X3,Y3,Z3) du repère (O,X,Y,Z),
  et
- des abscisses respectives X(TOG1), X(TOD1) des points TOG1, TOD1 dans la première occurrence (O1,X1,Y1,Z1 du repère (O,X,Y,Z) (les points TVG1, TVD1 étant respectivement confondus avec les points TOG1, TOD1),
  et/ou
- des abscisses respectives X(TOG2), X(TOD2) des points TOG2, TOD2 dans la seconde occurrence (O2,X2,Y2,Z2 du repère (O,X,Y,Z).

Ainsi, par exemple, si l'on effectue trois captures d'image dans les première, seconde et troisième postures, que les seconde et troisième occurrences correspondantes des angles de posture APIV2 et APIV3 sont égales et que les seconde et troisième occurrences D2, D3 de la distance D entre l'appareil de capture d'image 90 et l'origine O du repère (O,X,Y,Z) lié à la tête du porteur sont égales ou proches, on peut exploiter les seconde et troisième images capturées pour effectuer un calcul simple et précis du rayon d'oeil. Si l'on note respectivement ROG, ROD les rayons des yeux gauche et droit, le système de traitement et de caicui 93 peut exécuter les formules suivantes :

$$ROG = Abs(X(RCG2)-X(RCG3)) / OMEGA$$

$$ROD = Abs(X(RCD2)-X(RCD3)) / OMEGA$$

$$OMEGA = Arctan(d(ART,PVO).Sin(APIV2) / (d(ART,PVO).(1-cos(APIV2))+D2)$$
$$+ Arctan((Abs(X96-X116)-d(ART,PVO).Sin(APIV2))/(d(ART,PVO)+D2-$$
$$d(ART,PVO).Cos(APIV2))$$

où:

X(RCG2), X(RCD2) sont respectivement les abscisses suivant l'axe X du repère (O,X,Y,Z) des reflets cornéens gauche et droit RCG, RCD en seconde posture,
X(RCG3), X(RCD3) sont respectivement les abscisses suivant l'axe X du repère (O,X,Y,Z) des reflets cornéens gauche et droit RCG, RCD en troisième posture,
X96 et X116 sont respectivement les abscisses suivant l'axe X des points de visée 96 et 116 et
d(ART,PVO) est la distance entre l'axe de rotation vertical de la tête ART et le plan vertical monture PVO.

Pour terminer, le système de traitement et de calcul 93 déduit de la distance entre chaque lentille 100, 101 et l'oeil OD, OG correspondant et de l'orientation de chaque lentille, la configuration du référentiel de la lentille correctrice à réaliser par rapport aux yeux du porteur.

Autres exemples de variantes

On peut par exemple envisager de réaliser la mesure de la position du CRO de l'oeil du porteur en utilisant une source lumineuse infra-rouge à proximité de ou confondue avec la source lumineuse visible formant point de visée pour le regard du porteur. La mesure dans l'infra-rouge permet d'augmenter le contraste de la pupille de l'oeil et du reflet cornéen sur les images capturées et assure une meilleur précision de la mesure.

On peut également envisager de calculer la position moyenne du CRO de l'oeil du porteur en moyennant les mesures de la position du CRO de l'oeil du porteur sur plusieurs images capturées. L'utilisation d'une méthode mathématique connue comme la méthode des moindres carrés permet alors de minimiser les erreurs de mesure de cette position.

On pourra en particulier mesurer, au moyen d'un procédé analogue à celui qui vient d'être décrit, la position d'un point remarquable de chaque oeil autre que le centre de rotation de cet oeil, comme par exemple le centre de la pupille ou de l'iris, un point de la sclérotique adjacent à la commissure de la paupière ou au congé supérieur ou inférieur de la paupière.

D'autre part, dans l'exemple illustré par les figures 9 et 10, les premier et second points de visée (en première et seconde posture) sont distincts puisque les première et seconde postures sont distinctes. On pourrait cependant prévoir que les premier et second points de visée soient confondus, le porteur observant, dans les première et seconde postures, un seul et même point de visé tel que par exemple le centre d'une cible lumineuse ou non fixé sur un mur ou reposant sur une table ou un piètement.

On pourra par ailleurs avantageusement prévoir qu'au moins deux postures et deux points de visée sont tels que, lors de la capture d'image dans l'une des postures, les directions du regard des yeux correspondent à la vision de loin et que, lors de la capture d'image dans une autre posture, les directions du regard des yeux correspondent à la vision de près. Il est ainsi possible d'effectuer, à partir des images ainsi capturées, des données supplémentaires telles que les écarts pupillaires et hauteurs d'oeil en vision de loin et en vision de près, ou encore des informations sur le comportement du porteur (par exemple sa tendance à bouger davantage les yeux ou la tête pour passer de la vision de loin à la vision de près ou inversement).

On peut aussi prévoir que les différentes postures relatives de la tête du porteur par rapport à l'appareil de capture d'image soient prédéterminées. La position de l'appareil de capture d'image est alors fixe, ou en tout cas, prédéfinie pour chacune des captures d'image et le porteur est incité, pour chaque capture d'image, à placer sa tête dans la posture voulue et prédéfinie, au moyen d'un accessoire d'aide au positionnement quelconque. Cet accessoire peut par exemple consister en un viseur ou un pointeur LASER équipant la monture ou le système de repérage associé à une cible de position connue, que l'on demande au porteur de pointer, ou encore un dispositif repose-tête immobilisant la tête du porteur dans une posture prédéfinie. Dans ce cas, l'acquisition des différentes valeurs du paramètre de posture (tel que l'angle APIV) est globale : les différentes postures utilisées pour la mesure sont communes à tous les porteurs et les paramètres de posture correspondants sont donc des paramètres globaux (ou "variables globales" en langage informatique) qui ne font pas l'objet d'une nouvelle acquisition à chaque mesure.

On peut par ailleurs prévoir que le dispositif et le procédé soient conçus pour fonctionner pour une orientation du plan de Francfort qui n'est pas parallèle au plan horizontal PH du repère terrestre. Il est alors possible de mettre en oeuvre le procédé décrit ci-dessus, mais en considérant que la direction verticale est définie relativement au porteur et non pas par rapport au repère terrestre. Autrement dit, la direction verticale est alors définie comme étant la direction perpendiculaire à l'axe primaire de regard du porteur et contenue dans le plan sagittal PSAG. Le plan horizontal, perpendiculaire à la direction verticale, est alors défini comme étant confondu avec le plan de Francfort.

Le procédé décrit peut également être appliqué dans le cas d'une paire de lunettes de type percée. Dans ce cas, chaque baguette est directement fixée sur la lentille de présentation correspondante. Les calculs ou mesures réalisés relativement aux cercles (géométrie, orientation) dans la description ici-dessus sont alors réalisés relativement aux lentilles de présentation montées sur la monture de type percée.

En variante, pour une monture de type cerclée, on peut prévoir de mettre en oeuvre le procédé avec une monture cerclée dépourvue de lentilles de présentation. Dans ce cas, les tiges portant les billes d'appui prennent elles-même appui contre les cercles de la monture. On peut alors définir globalement pour chaque cercle son plan moyen passant par la position de la croix de montage. On exécute alors le procédé précédemment décrit en recherchant l'orientation de ce plan.

Toujours dans le cas d'une monture de type cerclée, il est possible de ne prévoir qu'une lentille de présentation gauche ou droite, en considérant que la configuration du référentiel de l'autre lentille est obtenue par symétrie par rapport au plan de symétrie de la monture. Dans ce cas, il est possible de garder les deux éléments de repérage associés aux deux yeux pour déterminer un éventuel angle de pivotement. Il est également possible de ne prévoir qu'un seul élément de repérage associé à un des deux yeux, en supposant que la tête est bien droite et que le plan de symétrie de la monture est bien confondu avec le plan sagittal.

On peut également envisager que le porteur ne garde pas la tête droite dans les première et deuxième postures relatives. Il tourne alors la tête autour d'un axe perpendiculaire au plan sagittal PSAG. Les paramètres de postures à déterminer comportent alors un angle de rotation autour de cet axe horizontal correspondant à un angle entre l'appareil de capture d'image et la tête du sujet dans un plan vertical.

Enfin, l'ordre d'exécution des étapes n'est pas limitatif et l'homme du métier saura le modifier à sa guise tout en préservant la cohérence d'ensemble du procédé.

## Revendications

1. Procédé de mesure de la position, suivant une direction horizontale du plan sagittal (PSAG), du <u>centre de rotation</u> (CROD, CROG) d'un oeil (OD, OG) d'un sujet dans un référentiel (O,X,Y,Z) lié à la tête de ce sujet, comportant les étapes de :

    S1) agencer dans une première posture relative (O1,X1,Y1,Z1) la tête du sujet par rapport à la pupille d'entrée (95) d'un appareil de capture d'image (90) disposé en regard du visage du sujet,
    S2) dans cette première posture relative, capturer une première image plane de l'oeil au moyen de l'appareil de capture d'image (90),
    S3) identifier, sur cette première image, l'image d'un premier point de référence prédéterminé (RCG1, RCD1) de l'oeil,
    S4) agencer dans une seconde posture relative (O2,X2,Y2,Z2) la tête du sujet par rapport à la pupille d'entrée (95) de l'appareil de capture d'image (90), distincte de la première posture relative (O1,X1,Y1,Z1),
    S5) dans cette seconde posture relative, capturer une seconde image plane de l'oeil au moyen de l'appareil de capture d'image (90),
    S6) identifier, sur cette seconde image, l'image d'un second point de référence prédéterminé (RCG2, RCD2) de l'oeil,
    S9) calculer ladite position du <u>centre de rotation</u> (CROD, CROG) de l'oeil en fonction des images des premier et second points de référence (RCG1, RCD1, RCG2, RCD2) de l'oeil et de première et seconde valeurs (APIV1, APIV2) d'un paramètre de posture (APIV) respectivement associées aux première et seconde postures relatives. <u>selon lequel, lors des première et seconde captures d'images, l'oeil regarde respectivement des premier et second points de visée (92, 92 ; 92, 116) ayant des positions connues l'une par rapport à l'autre.</u>
    <u>lesdites positions des premiers et seconds points de visée (92, 92 ; 92, 116) sont distinctes par rapport à la pupille (95) de l'appareil de capture d'image (90), les première et seconde postures et les premier et second points de visée étant tels que lors des première et seconde captures d'images, les directions correspondantes</u>

du regard de l'oeil sont distinctes dans le référentiel lié à la tête du sujet (O,X,Y,Z) et le calcul de la position du centre de rotation CROD, CROG) de l'oeil du sujet dans le référentiel lié au sujet étant de plus fonction des positions relatives des points de visée (92, 92, 92, 116) et

les valeurs du paramètre de posture sont calculées selon les étapes suivantes :

- on dispose sur la tête du sujet un élément de repérage (60, 70, 80 : 700 : 800) ayant au moins une caractéristique géométrique connue,
- chacune des première et seconde images planes capturées dans chaque posture relative au moyen de l'appareil de capture d'image intègre une image plane d'un élément de repérage (60, 70, 80 ; 700 ; 800) sur la tête du porteur, qui est traitée pour en mesurer une caractéristique géométrique dépendant d'une caractéristique géométrique connue de cet élément de repérage.
- les différentes valeurs du paramètre de posture relative (APIV) pour les différentes postures sont calculées en fonction de cette caractéristique géométrique mesurée de l'image capturée de l'élément de repérage et de la caractéristique géométrique connue de l'élément de repérage.

**2.** Procédé selon la revendication précédente, dans lequel, étant définies, dans la première posture relative, une première droite d'observation (DOG1, DOD1) reliant la pupille (95) de l'appareil de capture d'image (90) et le premier point de référence (RCG1, RCD1) de l'oeil et, dans la seconde posture relative, une seconde droite d'observation (DOG2, DOD2) reliant la pupille (95) de l'appareil de capture d'image (90) et le second point de référence (RCG2, RCD2) de l'oeil, les première et seconde postures relatives sont telles que ces première et seconde droites d'observation forment chacune avec leurs projections sur le plan sagittal (PSAG) et sur le pian de Francfort (PF) du sujet des angles respectifs inférieurs à 45 degrés.

**3.** Procédé selon l'une des revendications précédentes, dans lequel, étant définies, dans la première posture relative, une première droite d'observation (DOG1, DOD1) reliant la pupille (95) de l'appareil de capturé d'image (90) et le premier point de référence (RCG1, RCD1) de l'oeil et, dans la seconde posture relative, une seconde droite d'observation (DOG2, DOD2) reliant la pupille (95) de l'appareil de capture d'image (90) et le second point de référence (RCG2, RCD2) de l'oeil, les première et seconde postcures relatives sont telles que ces première et seconde droites d'observation présenter des configurations distinctes l'une de l'autre par rapport à la tête du sujet.

**4.** Procédé selon l'une des revendications précédentes, comportant de plus les étapes de :

S7) calculer, dans la première posture relative (O1,X1,Y1,Z1), une première droite d'observation (DOG1, DOD1) reliant la pupille (95) de l'appareil de capture d'image (90) et le premier point de référence (RCG1, RCD1) de l'oeil et, dans la seconde posture relative, une seconde droite d'observation (DOG2, DOD2) reliant la pupille (95) de l'appareil de capture d'image (90) et le seconde point de référence (RCG2, RCD2) de l'oeil,
S8) vérifier si ces première est seconde droites d'observation (DOG1, DOD1, DOG2, DOD2) présentent des configurations sensiblement distinctes l'une de l'autre par rapport à la tête du sujet et, dans la négative, exécuter à nouveau les étapes S1 à S3 ou les étapes S4 à S6.

**5.** Procédé selon l'une des revendications précédentes, dans lequel au moins deux postures et deux points de visée sont tels que, lors de la capture d'image dans l'une des postures, les directions du regard des yeux correspondent à la vision de loin et que, lors de la capture d'image dans une autre posture, les directions du regard des yeux correspondent à la vision de près.

**6.** Procédé selon l'une des revendications 1 à 5, dans lequel, lors au moins de la première ou seconde capture d'image, l'oeil regard respectivement un premier ou second point de visée (92j, ayant une position connue par rapport à la pupille de l'appareil de capture d'image (90) et dans lequel on exécute les étapes suivantes :

S10) agencer dans une troisième posture relative (O3,X3,Y3,Z3) la tête du sujet par rapport à la pupille d'entrée (95) de l'appareil de capture d'image (90), identique ou distincte des première et seconde postures relatives, l'oeil regardant dans cette troisième posture un troisième point de visée (116) ayant une position qui est connue par rapport à la pupille (95) de l'appareil de capture d'image (90) et qui est, par rapport au référentiel lié à la tête du sujet (O,X,Y,Z), distincte de celle du premier ou second point de visée (92),
S11) dans cette troisième posture relative (O3,X3,Y3,Z3), capturer une troisième image plane de l'oeil au moyen de l'appareil de capture d'image (90),
S12) identifier, sur cette troisième image, l'image d'un troisième point de référence prédéterminé (RCG3, RCD3) de l'oeil,

S13) calculer le rayon (ROG, ROD) de l'oeil en fonction :

- des images du premier ou second point de référence (RCG1, RCD1, RCG2, RCD2) et du troisième point de référence (RCG3, RCD3) de l'oeil,
- de la première ou seconde valeur (APIV1, APIV2) du paramètre de posture et d'une troisième valeur (APIV3) du paramètre de posture (APIV) associée à la troisième posture et
- des positions connues des points de visée (92, 116) par rapport à la pupille (95) de l'appareil de capture d'image (90),

le groupe d'étapes S10, S11, S12 étant, soit distinct du groupe d'étapes S1, S2, S3 d'une part et du groupe d'étapes S4, S5, S6 d'autre part, soit confondu avec l'un de ces groupes d'étapes.

7. Procédé selon l'une des revendications précédentes, dans lequel

- le premier point de référence (RCG1, RCD1) de l'oeil est le reflet d'une première source lumineuse (92) sur la cornée de l'oeil, cette première source lumineuse ayant une première position connue par rapport à la pupille d'entrée (95) de l'appareil de capture d'image (90),
- le second point de référence (RCG2, RCD2) de l'oeil est le reflet d'une seconde source lumineuse (116) sur la cornée de l'oeil, cette seconde source lumineuse étant distincte de ou confondue.avec la première source lumineuse et ayant une position connue par rapport à la pupille d'entrée (95) de l'appareil de capture d'image (90),
- le calcul de la position du centre de rotation (CROD, CROG) de l'oeil est réalisé en fonction, de plus, des positions des première et seconde sources lumineuses (92, 116).

8. Procédé selon les revendications 1 et 7 prises conjointement, dans lequel, lors des première et seconde captures d'images, l'oeil regarde respectivement la première et la seconde source lumineuse (92, 116), qui constituent ainsi lesdits premier et second points de visée.

9. Procédé selon l'une des revendications précédentes, dans lequel, pour calculer la position du centre de rotation (CROD, CROG) de l'oeil à l'étape S9, on exécute les sous-étapes de :

- déduire, de l'image du premier point de référencé (RCG1, RCD1) de l'oeil et de la première valeur (PSAG1) du paramètre de posture, les coordonnées, dans ledit référentiel de la tête du sujet (O,X,Y,Z), d'une première droite d'observation (DOG1, DOD1) reliant la pupille (95) de l'appareil de capture d'image (90) et le premier point de référence (RCG1, RCD1) de l'oeil,
- déduire, de l'image du second point de référence (RCG2, RCD2) de l'oeil et de la seconde valeur (PSAG2) du paramètre de posture, les coordonnées, dans ledit référentiel de la tête du sujet, d'une seconde droite d'observation (DOD2, DOG2) reliant la pupille (95) de l'appareil de capture d'image et le second point de référence de l'oeil (RCG2, RCD2),
- calculer la position du centre de rotation de l'oeil du sujet dans le référentiel lié à la tête du sujet en fonction des coordonnées des premières et secondes droites d'observation (DOG1, DOD1, DOD2, DOG2).

10. Procédé selon la revendication précédente, dans lequel la position du centre de rotation est calculée en tant que position du point d'intersection ou, si ces droites ne sont pas rigoureusement sécantes, de plus grande proximité des deux droites d'observation (DOG1, DOD1, DOD2, DOG2).

11. Procédé selon l'une des revendications précédentes, dans lequel ledit au moins un paramètre de posture comporte un ou plusieurs des paramètres suivants :

- l'angle horizontal entre l'appareil de capture d'image et la tête du sujet,
- l'angle vertical entre l'appareil de capture d'image et la tête du sujet,
- la distance entre l'appareil de' capture d'image et la tête du sujet.

12. Procédé selon l'une des revendications précédentes, dans lequel les premier et second points de référence de l'oeil se confondent en un même point de cet oeil consistant en l'un des points suivants de l'oeil :

- le centre de la pupille ou de l'iris,
- un point de la sclérotique adjacent à la commissure de la paupière ou au congé supérieur ou inférieur de la paupière.

**13.** Procédé selon l'une des revendications précédentes, dans lequel les premier et second points de référence de l'oeil sont des points de cet oeil distincts l'un de l'autre.

**14.** Procédé selon l'une des revendications 1 à 13,dans lequel lors d'au moins l'une des captures d'image, le sujet est équipé d'une monture de lunettes sur laquelle est monté l'élément de repérage (60, 70, 80 , 700 ; 800) et dans lequel on calcule, en fonction de cette caractéristique géométrique mesurée de l'image capturée de l'élément de repérage et de la caractéristique géométrique connue de l'élément de repérage, au moins une composante de l'orientation d'une lentille montée sur cette monture.

**15.** Procédé selon l'une des revendications précédentes, dans lequel, dans les étapes S1) et S4), on agence la tête du sujet par rapport à la pupille d'entrée de l'appareil de capture d'image (90) dans les première et seconde postures relatives, de manière que la position relative de la pupille (95) de l'appareil de capture d'image (90) par rapport à un axe de rotation vertical (ART) de la tête du sujet, ne soit pas modifiée entre lesdites première et seconde postures par un déplacement transversal de plus de 200 millimètres suivant une direction perpendiculaire à l'axe optique de l'appareil de capture d'image, et de manière que le sujet pivote la tête autour dudit axe de rotation vertical d'au moins 5 degrés et d'au plus 60 degrés entre lesdites première et seconde postures pour fixer du regard respectivement des premier et second points de visée ayant des positions distinctes connues l'une par rapport à l'autre.

## Claims

**1.** A method of measuring the position, in a horizontal direction of the sagittal plane (PSAG), of the center of rotation (CROD, CROG) of an eye (OD, OG) of a subject in a frame of reference (O,X,Y,Z) associated with the head of said subject, the method comprising the steps of:

S1) arranging the subject's head in a first relative posture (O1,X1,Y1,Z1) relative to the entry pupil (95) of an image capture appliance (90) placed facing the subject's face;
S2) in said first relative posture, capturing a first plane image of the eye by means of the image capture appliance (90);
S3) in said first image, identifying the image of a first predetermined reference point (RCG1, RCD1) of the eye;
S4) arranging the subject's head in a second relative posture (O2,X2,Y2,Z2) relative to the entry pupil (95) of the image capture appliance (90), the second relative posture being distinct from the first relative posture (O1,X1,Y1,Z1;
S5) in the second relative posture, capturing a second plane image of the eye by means of the image capture appliance (90);
S6) in said second image identifying the image of a second predetermined reference point (RCG2, RCD2) of the eye; and
S9) calculating said position of the center of rotation (CROD, CROG) of the eye as a function of the images of the first and second reference points (RCG1, RCD1, RCG2, RCD2) of the eye and of the first and second values (APIV1, APIV2) of a posture parameter (APIV) associated respectively with the first and second relative postures, wherein, while capturing the first and second images, the eye looks respectively at first and second sighting points (92, 92; 92, 116) having positions that are known relative to each other, said positions of the first and second sighting points (92, 92; 92, 116) relative to the pupil (95) of the image capture appliance (90) are distinct, the first and second postures and the first and second sighting points being such that, when capturing the first and second images, the corresponding directions of the gaze of the eye are distinct in the frame of reference associated with the subject's head (O,X,Y,Z) and the position of the center of rotation (CROD, CROG) of the subject's eye in the frame of reference associated with the subject is calculated also as a function of the relative positions of the sighting points (92, 92; 92, 116), and the values of the posture parameter are calculated using the following steps:

• placing a position-identification element (60, 70, 80; 700; 800) on the head of the subject, said element having at least one known geometrical characteristic;
• each of the first and second plane images captured in each relative posture by means of the image capture appliance incorporates a plane image of a position-identification element (60, 70, 80; 700; 800) on the wearer's head, with said image being processed to measure a geometrical characteristic thereof depending on a known geometrical characteristic of the position-identification element; and
• calculating the different values of the posture parameter (APIV) for the different postures as a function of said measured geometrical characteristic of the captured image of the position-identification element and of the known geometrical characteristic of the position-identification element.

2. A method according to the preceding claim, wherein, a first observation straight line (DOG1, DOD1) connecting the pupil (95) of the image capture appliance (90) and the first reference point (RCG1, RCD1) of the eye is defined in the first relative posture, a second observation straight line (DOG2, DOD2) connecting the pupil (95) of the image capture appliance (90) and the second reference point (RCG2, RCD2) of the eye is defined in the second relative posture, and the first and second relative postures are such that these first and second observation straight lines form respective angles of less than 45 degrees relative to their projections onto the sagittal plane (PSAG) and onto the Frankfort plane (PF).

3. A method according to any preceding claim, wherein, a first observation straight line (DOG1, DOD1) connecting the pupil (95) of :he image capture appliance (90) and the first reference point (RCG1, RCD1) of the eye is defined in the first relative posture, a second observation straight line (DOG2, DOD2) connecting the pupil (95) of the image capture appliance (90) and the second reference point (RCG2, RCD2) of the eye is defined in the second relative posture, and the first and second relative postures are such that these first and second observation straight lines present mutual distinct configurations relative to the subject's head.

4. A method according to any preceding claim, further including the steps of:

S7) calculating, in the first relative posture (O1,X1,Y1,Z1), a first observation straight line (DOG1, DOD1) connecting the pupil (95) of the image capture appliance (90) and the first reference point (RCG1, RCD1) of the eye, and in the second relative posture, a second observation straight line (DOG2, DOD2) connecting the pupil (95) of the image capture appliance (90) and the second reference point (RCG2, RCD2) of the eye; and

S8) verifying whether the first and second observation straight lines (DOG1, DOD1, DOG2, DOD2) present configurations that are substantially distinct from each other relative to the subject's head, and if not, executing steps S1 to S3 or S4 to S6 again.

5. A method according to any preceding claim, wherein at least two postures and two sighting points are such that while the image is being captured in one of the postures, the gaze directions of the eyes correspond to far vision, and while the image is being captured in another posture, the gaze directions of the eyes correspond to near vision.

6. A method according to any one of claims 1 to 5, wherein, at least while capturing the first and second images, the eye looks respectively at a first or second sighting point (92) having a known position relative to the pupil of the image capture appliance (90), and wherein, the following steps are executed:

S10) arranging the subject's head in a third relative posture (O3,X3,Y3,Z3 relative to the entry pupil (95) of the image capture appliance (90), which third relative posture is identical to or distinct from the first and second relative postures, the eye in said third relative posture looking at a third sighting point (116) having a position that is known relative to the pupil (95) of the image capture appliance (90) and that, relative to the frame of reference associated with the subject's head (O,X,Y,Z) is distinct from the position of the first or second sighting point (92);

S11) in said third relative posture (03,X3,Y3,Z3), capturing a third plane image of the eye by means of the image capture appliance (90);

S12) identifying in said third image, the image of a third predetermined reference point (RCG2, RCD3) of the eye; and

S13) calculating the radius (ROG, ROD) of the eye as a function:

• of the images of the first or second reference points (RCG1, RCD1, RCG2, RCD2) and of the third reference point (RCG3, RCD3) of the eye;
• of the first or second value (APIV1, APIV2) of the posture parameter and a third value (APIV3) of the posture parameter (APIV) associated with the third posture; and
• of the known positions of the sighting points (92, 116) relative to the pupil (95) of the image capture appliance (90);

the group of steps S10, S11, S12 being either distinct from both of the groups of steps S1, S2, S3 and S4, S5, S6, or else coinciding with one of said groups of steps.

7. A method according to any preceding claim, wherein:

• the first reference point (RCG1, RCD1) of the eye is the reflection of a first light source (92) on the cornea of the eye, said first light source having a first position that is known relative to the entry pupil (95) of the image capture appliance (90);
• the second reference point (RCG2, RCD2) of the eye is the reflection of a second light source (116) on the cornea of the eye, said second light source being distinct from or coinciding with the first light source and having a position that is known relative to the entry pupil (95) of the image capture appliance (90); and
• the position of the center of rotation (CROD, CROG) of the eye is calculated as a function, also, of the positions of the first and second light sources (92, 116).

8. A method according to claims 6 and 7 taken together, wherein, while capturing the first and second images, the eye looks respectively at the first and at the second light sources (92, 116), thereby constituting said first and second sighting points.

9. A method according to any preceding claim, wherein, in order to calculate the position of the center of rotation (CROD, CROG) of the eye in step S9, the following substeps are executed:

• from the image of the first reference point (RCG1, RCD1) of the eye and from the first value (PSAG1) of the posture parameter, deducing the coordinates, in said frame of reference of the subject's head (O,X,Y,Z), of a first observation straight line (DOG1, DOD1) connecting the pupil (95) of the image capture appliance (90) and the first reference point (RCG1, RCD1) of the eye;
• from the image of the second reference point (RCG2, RCD2) of the eye and from the second value (PSAG2) of the posture parameter, deducing the coordinates in said frame of reference of the subject's head, of a second observation straight line (DOD2, DOG2) connecting the pupil (95) of the image capture appliance and the second reference point of the eye (RCG2, RCD2); and
• calculating the position of the center of rotation of the subject's eye in the frame of reference associated with the subject's head as a function of the coordinates of the first and second observation straight lines (DOG1, DOD1, DOD2, DOG2).

10. A method according to the preceding claim, wherein the position of the center of rotation is calculated as the position of the point of intersection of the two observation straight lines (DOG1, DOD1, DOD2, DOG2), or if said straight lines do not intersect accurately, the point where said observation straight lines come closest together.

11. A method according to any preceding claim, wherein said at least one posture parameter includes one or more of the following parameters:

• the horizontal angle between the image capture appliance and the subject's head;
• the vertical angle between the image capture appliance and the subject's head; and
• the distance between the image capture appliance and the subject's head.

12. A method according to any preceding claim, wherein the first and second reference points of the eye coincide at a single point of said eye, being constituted by one of the following points of the eye:

• the center of the pupil or of the iris; and
• a point of the sclera adjacent to the canthus or to the upper or lower edge of the eyelid.

13. A method according to any preceding claim, wherein the first and second reference points of the eye are points of said eye that are distinct from each other.

14. A method according to any one of claims 1 to 13, wherein while capturing at least one of the images, the subject is fitted with an eyeglass frame having mounted thereon the position-identification element (60, 70, 80; 700; 800), and wherein, as a function of said measured geometrical characteristic of the image captured of the position-identification element and of the known geometrical characteristic of the position-identification element, at least one component of the orientation of a lens mounted on said frame is calculated.

15. A method according to any preceding claim, wherein, in steps S1) and S4), the subject's head is arranged relative to the entry pupil of the image capture appliance (90) in the first and second relative postures in such a manner that the position of the pupil (95) of the image capture appliance (90) relative to a vertical axis of rotation (ART) of the subject's head is not modified between said first and second postures by a transverse movement of more than 200 millimeters in a direction perpendicular to the optical axis of the image capture appliance, and in such a manner that the subject pivots the head about said vertical axis of rotation through at least 5 degrees and not more than 60 degrees between said first and second postures in order to gaze respectively at the first and second sighting points that have different positions that are known relative to each other.

**Patentansprüche**

1. Messverfahren der Position des Drehpunkts (CROD, CROG) eines Auges (OD = rechtes Auge, OG = linkes Auge) einer Person entlang einer waagrechten Richtung der Sagittalebene (PSAG) in einem Referenzsystem (O, X, Y, Z) in Verbindung mit dem Kopf der Person mit folgenden Schritten:

   S1) Anordnen des Kopfes der Person in einer ersten relativen Stellung (O1, X1, Y1, Z1) in Bezug zur Eintrittspupille (95) eines Bildaufnahmegeräts (90), das gegenüber dem Gesicht der Person angeordnet ist,
   S2) Aufnehmen eines ersten planen Bildes des Auges in dieser ersten relativen Stellung mit einem Bildaufnahmegerät (90),
   S3) Identifizieren des Bildes eines ersten vorbestimmten Referenzpunktes (RCG1, RCD1) des Auges auf diesem ersten Bild,
   S4) Anordnen des Kopfes der Person in einer zweiten relativen Stellung (O2, X2, Y2, Z2) in Bezug zur Eintrittspupille (95) des Bildaufnahmegeräts (90), die sich von der ersten relativen Stellung (O1, X1, Y1, Z1) unterscheidet,
   S5) Aufnehmen eines zweiten planen Bildes des Auges in dieser zweiten relativen Stellung mit dem Bildaufnahmegerät (90),
   56) Identifizieren des Bildes eines zweiten vorbestimmten Referenzpunktes (RCG2, RCD2) des Auges auf diesem zweiten Bild,
   59) Berechnen der Position des Drehpunktes (CROD, CROG) des Auges in Abhängigkeit der Binder des ersten und zweiten Referenzpunktes (RCG1, RCD1, RCG2, RCD2) des Auges und von ersten und zweiten Werten (APIV1, APIV2) eines Stellungsparameters (APIV), der jeweils der ersten und zweiten relativen Stellung zugeordnet ist,
   gemäß dem das Auge während der ersten und zweiten Bildaufnahme jeweils auf erste und zweite Zielpunkte (92, 92; 92, 116) mit zueinander bekannten Positionen schaut, wobei sich die Positionen des ersten und zweiten Zielpunkts (92, 92; 92, 116) von der Eintrittspupille (95) des Bildaufnahmegeräts (90) unterscheiden, die erste und zweite Stellung sowie der erste und zweite Zielpunkt derart sind, dass sich bei der ersten unit zweiten Bildaufnahme die entsprechenden Richtungen des Blicks des Auges im Referenzsystem in Verbindung mit dem Kopf der Person (O, X, Y, Z) unterscheiden und die Berechnung der Position des Drehpunkts (CROD, CROG) des Auges der Person im Referenzsystem in Verbindung mit der Person zudem von den relativen Positionen der Zielpunkte (92, 92; 92, AA6) abhängt und
   die Werte des Stellungsparameters gemäß folgenden Schritten berechnet werden:

      - auf dem Kopf der Person bringt man ein Markierungselement (60, 70, 80; 700; 800) mit mindestens einem bekannten geometrischen Merkmal an,
      - jedes der ersten und zweiten mit dem Bildaufnahmegerät aufgenommenen planen Bilder in jeder relativen Stellung umfasst ein planes Bild eines Markierungselements (60, 70, 80; 700; 800) auf dem Kopf des Trägers, das bearbeitet wird, um ein geometrisches Merkmal zu messen, das von einem bekannten geometrischen Merkmal dieses Markierungselements abhängt,
      - die einzelnen Werte des Parameters der relativen Stellung (APIV) für die verschiedenen Stellungen werden in Abhängigkeit dieses gemessenen geometrischen Merkmals des aufgenommenen Bildes des Markierungselements und des bekannten geometrischen Merkmals des Markierungselements berechnet.

2. Verfahren nach vorausgehendem Anspruch, bei dem in der ersten relativen Stellung eine erste Beobachtungsgerade (DOG1, DOD1) definiert wird, die die Eintrittspupille (95) des Bildaufnahmegeräts (90) und den ersten Referenzpunkt (RCG1, RCD1) des Auges verbindet, und bei dem in der zweiten relativen Stellung eine zweite Beobachtungsgerade (DOG2, DOD2) definiert wird, die die Eintrittspupille (95) des Bildaufnahmegeräts (90) und den zweiten Referenzpunkt (RCG2, RCD2) des Auges verbindet, wobei die erste und zweite relative Stellung derart sind, dass diese erste und zweite Beobachtungsgerade jeweils mit ihren Projizierungen auf der Sagittalebene (PSAG) und auf der Frankfurtebene (PF) der Person entsprechende Winkel unter 45 Grad bildet.

3. Verfahren nach einem der vorausgehenden Ansprüche, bei dem In der ersten relativen Stellung eine erste Beobachtungsgerade (DOG1, DOD1) definiert ist, die die Eintrittspupille (95) des Bildaufnahmegeräts (90) und den ersten Referenzpunkt (RCG1, RCD1) des Auges verbindet, und bei dem in der zweiten relativen Stellung eine zweite Beobachtungsgerade (DOG2, DOD2) definiert ist, die die Eintrittspupille (95) des Bildaufnahmegeräts (90) und den zweiten Referenzpunkt (RCG2, RCD2) des Auges verbindet, wobei die erste und zweite relative Stellung derart sind, dass diese erste und zweite Beobachtungsgeraden zueinander unterschiedliche Konfigurationen in Bezug auf den Kopf der Person darstellen.

4. Verfahren nach einem der vorausgehenden Ansprüche, das zudem folgende Schritte umfasst:

S7) Berechnen einer ersten Beobachtungsgeraden (DOG1, DOD1), die die Eintrittspupille (95) des Bildaufnahmegeräts (90) und den ersten Referenzpunkt (RCG1, RDD1) des Auges in einer ersten relativen Stellung (O1, X1, Y1, Z1) verbindet, sowie einer zweiten Beobachtungsgeraden (DOG2, DOD2), die die Eintrittspupille (95) des Bildaufnahmegeräts (90) und den zweiten Referenzpunkt (RCG2, RCD2) des Auges in einer zweiten relativen Stellung verbindet,

S8) Überprüfen, ob diese erste und zweite Beobachtungsgerade (DOG1, DOD1, DOG2, DOD2) zueinander geringfügig unterschiedliche Konfigurationen in Bezug auf den Kopf der Person darstellen, und wenn nicht, die Schritte S1 bis S3 bzw. die Schritte S4 bis S6 erneut ausführen.

5. Verfahren nach einem der vorausgehenden Ansprüche, bei dem mindestens zwei Stellungen und zwei Zielpunkte derart sind, dass die Blickrichtungen der Augen, während das Bild in einer der Stellungen aufgenommen wird, der Fernsicht entsprechen und bei dem die - Blickrichtungen der Augen, während das Bild in der anderen Stellung aufgenommen wird, der Nahsicht entsprechen.

6. Verfahren nach einem der Ansprüche 1 bis 5, bei dem das Auge, während mindestens der ersten oder zweiten Bildaufnahme jeweils auf einen ersten oder zweiten Zielpunkt (92) mit einer in Bezug zur Eintrittspupille des Bildaufnahmegeräts (90) bekannten Position blickt und bei dem man folgende Schritte ausführt:

S10) Anordnen des Kopfes der Person in einer dritten relativen Stellung (O3, X3, Y3, Z3) in Bezug zur Eintrittspupille (95) des Bildaufnahmegeräts (90), die mit der ersten und zweiten relativen Stellung identisch ist oder sich von ihnen unterscheidet, wobei das Auge in dieser dritten Stellung auf einen dritten Zielpunkt (116) mit einer in Bezug zur Eintrittspupille (95) des Bildaufnahmegeräts (90) bekannten Position blickt, welche sich in Bezug zum Referenzsystem in Verbindung mit dem Kopf der Person (O, X, Y, Z) von der des ersten oder zweiten Zielpunkts (92) unterscheidet,

S11) Aufnehmen eines dritten planen Bildes des Auges in dieser dritten relativen Stellung (03, X3, Y3, Z3) mit dem Bildaufnahmegerät (90),

S12) Identifizieren des Bildes eines dritten vorbestimmten Referenzpunktes (RCG3, RCD3) des Auges auf diesem dritten Bild,

S13) Berechnen des Augenradius (ROG, ROD) in Abhängigkeit von:

- den Bildern des ersten oder zweiten Referenzpunktes (RCG1, RCD1, RCG2, RCD2) und des dritten Referenzpunktes (RCG3, RCD3) des Auges,
- dem ersten oder zweiten Wert (APIV1, APIV2) des Stellungsparameters und eines dritten Werts (APIV3) des Stellungsparameters (APIV), der der dritten Stellung zugeordnet ist, und
- den bekannten Positionen der Zielpunkte 92, 116) in Bezug auf die Eintrittspupille (95) des Bildaufnahmegeräts (90),

wobei die Gruppe der Schritte S10, S11, S12 sich entweder von der Gruppe der Schritte S1, S2, S3 einerseits und der Gruppe der Schritte S4, S5, S6 andererseits unterscheidet, oder mit einer dieser Gruppen von Schritten übereinstimmt.

7. Verfahren nach einem der vorausgehenden Ansprüche, bei dem

- der erste Referenzpunkt (RCG1, RCD1) des Auges die Spiegelung einer ersten Lichtquelle (92) auf der Augenhornhaut ist, wobei diese erste Lichtquelle eine in Bezug zur Eintrittspupille (95) des Bildaufnahmegeräts (90) bekannte erste Position hat,
- der zweite Referenzpunkt (RCG2, RGD2) des Auges die Spiegelung einer zweiten Lichtquelle (116) auf der Augenhornhaut ist, wobei sich diese zweite Lichtquelle von der ersten Lichtquelle unterscheidet oder mit ihr übereinstimmt und eine in Bezug zur Eintrittspupille (95) des Bildaufnahmegeräts (90) bekannte Position hat,
- die Berechnung der Position des Augendrehpunktes (CROD, CROG) zudem in Abhängigkeit der Positionen der ersten und zweiten Lichtquelle (92, 116) erfolgt.

8. Verfahren nach den Ansprüchen 1 und 7 (zusammengefasst), bei dem das Auge während der ersten und zweiten Bildaufnahme jeweils in die erste und zweite Lichtquelle (92, 116) schaut, die somit den ersten und zweiten Zielpunkt bilden.

9. Verfahren nach einem der vorausgehenden Ansprüche, bei dem man, um die Position des Augendrehpunkts (CROD, CROG) im Schritt S9 zu berechnen, folgende untergeordnete Schritte ausführt:

- Ableiten anhand des Bildes des ersten Referenzpunktes (RCG1, RCD1) des Auges und des ersten Wertes (PSAG1) des Stellungsparameters der Koordinaten im Referenzsystem für den Kopf der Person (O, X, Y, Z) einer ersten Beobachtungsgeraden (DOG1, DOD1), die die Eintrittspupille (95) des Bildaufnahmegeräts (90) und den ersten Referenzpunkt (RCG1, RCD1) des Auges verbindet,
- Ableiten anhand des Bildes des zweiten Referenzpunktes (RCG2, RCD2) des Auges und des zweiten Wertes (PSAG2) des Stellungsparameters der Koordinaten im Referenzsystem für den Kopf der Person einer zweiten Beobachtungsgeraden (DOD2, DOG2), die die Eintrittspupille (95) des Bildaufnahmegeräts und den zweiten Referenzpunkt (RCG2, RCD2) des Auges verbindet,
- Berechnen der Position des Augendrehpunkts der Person im Referenzsystem in Verbindung mit dem Kopf der Person, in Abhängigkeit der Koordinaten der ersten und zweiten Beobachtungsgeraden (DOG1, DOD1, DOD2, DOG2).

10. Verfahren nach vorausgehendem Anspruch, bei dem die Position des Drehpunkts als Position des Schnittpunkts, oder wenn sich diese Geraden nicht genau schneiden, als Position der größten Nähe der beiden Beobachtungsgeraden (DOG1, DOD1, DOD2, DOG2) berechnet wird.

11. Verfahren nach einem der vorausgehenden Ansprüche, bei dem der mindestens eine Stellungsparameter einen bzw. mehrere der folgenden Parameter umfasst:

- den waagrechten Winkel zwischen dem Bildaufnahmegerät und dem Kopf der Person,
- den senkrechten Winkel zwischen dem Bildaufnahmegerät und dem Kopf der Person,
- den Abstand zwischen dem Bildaufnahmegerät und dem Kopf der Person.

12. Verfahren nach einem der vorausgehenden Ansprüche, bei dem der erste und zweite Referenzpunkt des Auges in einem gleichen Punkt dieses Auges zusammentreffen, der einem der folgenden Punkte des Auges entspricht:

- dem Mittelpunkt der Pupille oder der Regenbogenhaut,
- einem Punkt der Lederhaut neben der Kommissur des Augenlids oder neben der oberen oder unteren Abrundung des Augenlids.

13. Verfahren nach einem der vorausgehenden Ansprüche, bei dem der erste und zweite Referenzpunkt des Auges zueinander unterschiedliche Punkte dieses Auges sind.

14. Verfahren nach einem der Ansprüche 1 bis 13, bei dem die Person während mindestens einer der Bildaufnahmen mit einem Brillengestell ausgestattet wird, auf dem das Markierungselement (60, 70, 80; 700; 800) montiert ist und bei dem man in Abhängigkeit dieses gemessenen geometrischen Merkmals des aufgenommenen Bildes des Markierungselements und des bekannten geometrischen Merkmals des Markierungselements mindestens eine Richtungskomponente eines in dieses Brillengestell montierten Brillenglases berechnet.

15. Verfahren nach einem der vorausgehenden Ansprüche, bei dem man den Kopf der Person in den Schritten S1) und S4) in Bezug zur Eintrittspupille des Bildaufnahmegeräts (90) in der ersten und zweiten relativen Stellung so anordnet, dass die relative Position der Eintrittspupille (95) des Bildaufnahmegeräts (S0) in Bezug zu einer senkrechten Drehachse (ART) des Kopfes der Person zwischen der ersten und zweiten Stellung nicht durch eine Querbewegung von über 200 Millimeter entlang einer zur optischen Achse des Bildaufnahmegeräts senkrechten Richtung geändert wird, sowie derart, dass die Person den Kopf um diese senkrechte Drehachse um mindestens 5 Grad und um höchstens 60 Grad zwischen der ersten und zweiten Stellung dreht, um den Blick jeweils auf den ersten und zweiten Zielpunkt mit zueinander unterschiedlichen und bekannten Positionen zu richten.

Fig.1

Y

13

14

OD

0

X

OG

CMD

Z

15

100

101

PLG

XLD

PLD

XLG

PSAG

PF

N90

AMV

GAMMA

ARV

Fig.2

20

ARHD
AMD

70

195 191

60

61

192 196 190

80

ARHG

AMG

71

81

TETA

25 23

26

29

27

193

28

TETA

37

30

40

38

22

21

A1

24

Fig.3

Fig.4

Fig.5

Fig.6

## Fig.7

## Fig.8

Fig.9

Fig.10

Fig.11

EP 2 134 249 B1

**EP 2 134 249 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006106248 A1
- DE 102004020356 A1
- EP 1747750 A1
- WO 0209025 A1